# EUROPEAN PATENT APPLICATION

(11) **EP 4 289 415 A1**
(43) Date of publication of application: **13.12.2023**
(21) Application number: 22750078.2
(22) Date of filing: 07.02.2022
(51) Int. Cl.: A61K 9/00, A61K 9/127, A61K 47/26, A61K 31/475, A61K 31/704, A61P 35/00

(54) **COMPOSITION FOR PENETRATING BLOOD-BRAIN BARRIER, CONTAINING SONOSENSITIVE LIPOSOMES AS ACTIVE INGREDIENTS**

(30) Priority: 08.02.2021 KR 20210017524; 04.02.2022 KR 20220015104
(71) Applicant: IMGT Co, Ltd., Seongnam-si, Gyeonggi-do 13605 (KR)
(72) Inventor: MOON, Hyungwon, Hwaseong-si Gyeonggi-do 18503 (KR); KIM, Yoonseok, Gwangju-si Gyeonggi-do 12820 (KR); JUNG, Eun Ah, Seoul 06277 (KR); KIM, Hyun Ryoung, Seongnam-si Gyeonggi-do 13581 (KR)
(74) Representative: Dehns
(86) International application number: PCT/KR2022/001847
(87) International publication number: WO 2022/169329

(57) **Abstract**

The present invention relates to sonosensitive liposomes for penetrating the blood-brain barrier, wherein the sonosensitive liposomes have excellent drug loading efficiency and ultrasound-induced drug release effect and can effectively penetrating the blood-brain barrier during ultrasound stimulation. Particularly, sonosensitive liposomes according to the present invention can circulate in the body for a long time, and thus has better blood-brain barrier penetration efficiency, and has a high affinity for brain tumor cells, and thus has excellent delivery to tumor sites. Therefore, sonosensitive liposomes according to the present invention can be used as a drug delivery system for delivering brain disease therapeutic agents and the like to the brain. Specifically, the present inventors discovered the optimum cavitation condition capable of stably opening the blood-brain barrier in order to further enhance drug delivery effects of the sonosensitive liposomes, and thus, if the cavitation condition is grafted onto the sonosensitive liposomes for penetrating the blood-brain barrier, according to the present invention, excellent treatment effects are expected to be obtained for various brain diseases.

## Description

### [Technical Field]

The present invention relates to a composition for penetrating blood-brain barrier, comprising sonosensitive liposome, and so on.

This application claims priority based on Korean Patent Application No. 10-2021-0017524 filed on February 8, 2021, and Korean Patent Application No. 10-2022-0015104 filed on February 4, 2022. All contents disclosed in the specifications and drawings of these applications are incorporated herein by reference.

### [Background Art]

As the incidence of brain tumors, brain infections caused by bacteria or viruses, and neurological disorders is increasing, the need for technologies that can precisely administer drugs to the brain is also growing. In particular, the most commonly performed treatment for brain tumors currently is surgical treatment, which involves opening the skull and removing the tumor.

However, opening the skull not only puts stress on the patient, but also raises concerns about the side effects of nerve cell damage during surgery. Nevertheless, surgical treatment is used because drug delivery is inhibited by the blood-brain barrier (BBB), even when using drugs that target the brain. Therefore, anticancer drugs used for the treatment of brain diseases such as brain tumors are primarily limited to formulations that can penetrate the blood-brain barrier, but these too have difficulty achieving the delivery effect accurately to the disease site, making it difficult to achieve complete therapeutic effect. Accordingly, there is an urgent need for the development of technology that can accurately deliver drugs to brain tissues.

Recently, researches on technology to deliver drugs to the brain using ultrasound have been carried out globally. Cavitation caused by ultrasound and microbubbles, which are ultrasound contrast agents, can temporarily open the blood-brain barrier, and are being applied in the treatment of brain diseases as a means of drug delivery. To this end, after injecting microbubbles and focusing ultrasound in 3D, the ultrasound is precisely emitted at the brain tumor site, causing the microbubbles located near the blood-brain barrier to locally open the blood-brain barrier, and thereby delivering the drug to the brain tissue and triggering the mechanism for treating brain diseases. However, the currently used anticancer drugs are small molecule chemotherapeutics that show limitations in delivery effect to the brain due to rapid excretion out of the body after administration and fast absorption into normal tissues.

Therefore, by developing a drug delivery carrier that has high blood-brain barrier penetration efficiency and can circulate in the blood for a long time, and applying it together with ultrasound and microbubbles, drugs can be delivered more effectively to the brain.

### [Disclosure]

### [Technical Problem]

As a result of the research to solve the above-mentioned problems, the present inventors have developed sonosensitive liposomes (IMP302), which can not only effectively pass through the blood-brain barrier, but also circulate in the body for a long time, thereby further enhancing blood-brain barrier penetration efficiency, and having high affinity for brain tumor cells, maximizing the effect of drug delivery to the tumor site.

Thus, an object of the present invention is to provide a composition for penetrating the blood-brain barrier, comprising the sonosensitive liposomes as active ingredients.

Another object of the present invention is to provide a drug delivery carrier for penetrating the blood-brain barrier, comprising the sonosensitive liposomes.

Still another object of the present invention is to provide a pharmaceutical composition for preventing or treating of brain diseases, comprising the sonosensitive liposomes.

Yet another object of the present invention is to provide a method for manufacturing the sonosensitive liposomes for penetrating the blood-brain barrier.

However, technical problems to be solved in the present invention are not limited to the above-described problems, and other problems which are not described herein will be fully understood by those of ordinary skill in the art from the following descriptions.

### [Technical Solution]

The present invention provides a composition for penetrating the blood-brain barrier, comprising sonosensitive liposomes as active ingredients. The sonosensitive liposomes comprise DSPC (1,2-Distearoyl-sn-glycero-3-phosphocholine), DSPE-mPEG2000 (1,2-Distearoyl-sn-glycero-3-phosphoethanolamine-N-[methoxy(polyethylene glycol)-2000]), DOPE (1,2-Dioleoyl-sn-glycero-3-phosphoethanolamine), cholesterol, and lyso-PC (lysophosphatidylcholine). The composition comprises a pharmaceutical composition.

In one embodiment of the present invention, the DSPC, DSPE-mPEG2000, DOPE, cholesterol, and lyso-PC can be included in a molar ratio (mole%) of 1~50 : 1∼10 : 5~80 : 0.1∼50 : 0.1-20, but is not limited thereto.

In another embodiment of the present invention, the DSPC can be included at 0.1 to 50 dry weight% based on the total liposome,
the DSPE-mPEG2000 can be included at 3 to 50 dry weight% based on the the total liposome,
the DOPE can be included at 1 to 80 dry weight% based on the total liposome,
the cholesterol can be included at 0.05 to 40 dry weight% based on the total liposome, and
the lyso-PC can be included at 0.5 to 10 dry weight% based on the total liposome, but is not limited thereto.

In yet another embodiment of the present invention, the sonosensitive liposomes can further comprise at least one selected from a group consisting of sphingolipid and polysorbate, but is not limited thereto.

Furthermore, the present invention provides a composition for penetrating the blood-brain barrier, which comprises sonosensitive liposomes comprising DOPE (1,2-Dioleoyl-sn-glycero-3-phosphoethanolamine) and sphingolipid as active ingredients.

In another embodiment of the present invention, the DOPE can be included in a molar ratio (%) of 5 to 80 based on the total liposome, and the sphingolipid can be included in a molar ratio (%) of 5 to 80 based on the total liposome, but is not limited thereto.

In still another embodiment of the present invention, the sonosensitive liposomes may further comprise DSPE-mPEG2000, but not limited thereto.

In yet another embodiment of the present invention, DSPE-mPEG2000 may be included in a molar ratio (%) of 1 to 20 based on the total liposome, but not limited thereto.

In yet another embodiment of the present invention, the sonosensitive liposomes may satisfy at least one of the following features selected from the group consisting of, but not limited to:
(a) a particle size of 100 to 200 nm; and
(b) the ratio of the drug loaded in the liposome based on the total drug added is 50 to 100%.

In yet another embodiment of the present invention, the sonosensitive liposomes may be capable of crossing the blood-brain barrier when exposed to ultrasound, but not limited thereto.

In yet another embodiment of the present invention, the composition may be for delivering a drug to the brain, but not limited thereto.

In yet another embodiment of the present invention, the drug may be a therapeutic agent for brain disease, but not limited thereto.

In yet another embodiment of the present invention, the brain disease may be at least one selected from the group consisting of brain tumors, brain infections caused by bacteria or viruses, Parkinson's disease, encephalitis, stroke, palsy, Alzheimer's, Lou Gehrig's disease, Huntington's disease, Pick's disease, Creutzfeldt-Jakob disease, epilepsy, thrombosis, embolism, brain infarction, small artery occlusion, and cerebral metabolic disorders, but not limited thereto.

In yet another embodiment of the present invention, the therapeutic agent for brain disease may comprise at least one selected from the group consisting of vincristine, vinblastine, vinflunine, vindesine, vinorelbine, temozolomide, carmustine, lomustine, cabazitaxel, docetaxel, larotaxel, ortataxel, paclitaxel, tesetaxel, ixabepilone, lomustine, procarbazine, rituximab, tocilizumab, temozolomide, carboplatin, erlotinib, irinotecan, enzastaurin, vorinostat, doxorubicin, cisplatin, Gleevec, 5-fluorouracil, tamoxifen, topotecan, belotecan, imatinib, floxuridine, gemcitabine, leuprolide, flutamide, zoledronate, methotrexate, camptothecin, hydroxyurea, streptozocin, valrubicin, retinoic acid, mechlorethamine, chlorambucil, busulfan, doxifluridine, mitomycin, prednisone, Afinitor, mitoxantrone, levodopa, carbidopa, entacapone, tolcapone, dopamine agonists, donepezil, galantamine, rivastigmine, memantine, anticholinergics, and amantadine, but is not limited thereto.

In yet another embodiment of the present invention, the sonosensitive liposomes may be hydrated with ammonium sulfate, ammonium citrate, or TEA-SOS, but is not limited thereto.

In yet another embodiment of the present invention, the composition may be administered sequentially or simultaneously with ultrasound treatment, but is not limited thereto.

In yet another embodiment of the present invention, the ultrasound may satisfy at least one of the following features, but is not limited thereto:
(a) The frequency of the ultrasound is from 20 kHz to 3 MHz; and
(b) The duty cycle is from 0.5 to 20%.

In yet another embodiment of the present invention, the ultrasound treatment may occur sequentially or simultaneously with the administration of microbubbles, but is not limited thereto.

Moreover, the present invention provides a drug delivery carrier for penetrating the blood-brain barrier, comprising sonosensitive liposomes as active ingredients. The sonosensitive liposomes comprises DSPC (1,2-Distearoyl-sn-glycero-3-phosphocholine), DSPE-mPEG2000 (1,2-Distearoyl-sn-glycero-3-phosphoethanolamine-N-[methoxy(polyethylene glycol)-2000]), DOPE (1,2-Dioleoyl-sn-glycero-3-phosphoethanolamine), cholesterol, and lyso-PC (lysophosphatidylcholine).

In addition, the present invention provides a drug delivery carrier for penetrating the blood-brain barrier, comprising sonosensitive liposomes as active ingredients. The sonosensitive liposomes comprises DOPE (1,2-Dioleoyl-sn-glycero-3-phosphoethanolamine) and sphingolipid.

Furthermore, the present invention provides a method for producing the composition for penetrating the blood-brain barrier, which comprises the following steps:
(S1) dissolving at least one selected from the group consisting of DSPC, DSPE-mPEG2000, DOPE, cholesterol, and lyso-PC in a first organic solvent;
(S2) evaporating the organic solvent to manufacture a liposome film; and
(S3) hydrating the liposome film with an aqueous solution.

In one embodiment of the present invention, the first organic solvent in step (S1) may be at least one selected from the group consisting of dimethylacetamide, dimethylformamide, dimethylsulfoxide, chloroform, methanol, ethanol, and ether, but is not limited thereto.

In yet another embodiment of the present invention, the first organic solvent in step (S1) may be supplemented with polysorbate, but is not limited thereto.

In addition, the present invention provides a pharmaceutical composition for preventing or treating of brain disease, which comprises the sonosensitive liposomes as active ingredients. Preferably, the sonosensitive liposomes contain a drug.

In addition, the present invention provides a method for preventing or treating brain diseases, which comprises a step of administering an effective dose of the sonosensitive liposomes to a subject in need. Preferably, the method may further comprise a step of processing ultrasound and/or administering microbubbles.

Moreover, the present invention provides the use of the sonosensitive liposomes for the preventing or treating of brain diseases.

Moreover, the present invention provides the use of the composition for the manufacture of a therapeutic agent for brain disease.

Furthermore, the present invention provides a method for delivering a drug to the brain, which comprises a step of administering an effective dose of the drug-loaded sonosensitive liposomes to a subject in need. Preferably, the method may further comprise a step of processing ultrasound and/or administering microbubbles.

Furthermore, the present invention provides the use of the sonosensitive liposomes for drug delivery to the brain.

Furthermore, the present invention provides the use of the sonosensitive liposomes for the manufacture of a drug delivery carrier targeting the brain.

### [Advantageous Effects]

The present invention relates to sonosensitive liposomes for penetrating the blood-brain barrier. The sonosensitive liposome not only demonstrates superior drug encapsulation efficiency and drug release effects under ultrasound stimulation, but also effectively penetrates the blood-brain barrier when stimulated by ultrasound. Particularly, the sonosensitive liposomes according to the present invention can maintain long-term circulation in the body, leading to excellent blood-brain barrier penetration efficiency, and have high affinity for brain tumor cells, thereby offering outstanding delivery effect to tumor sites. Therefore, the sonosensitive liposomes according to the present invention can be utilized as a drug delivery carrier for delivering a therapeutic agent for brain disease to the brain. Specifically, the inventors of the present invention have identified optimal cavitation conditions that can stably open the blood-brain barrier, with the aim of further enhancing the drug delivery effect of the sonosensitive liposomes. It is anticipated that excellent therapeutic effects can be obtained in various brain diseases by combining these cavitation conditions together with sonosensitive liposomes for penetrating the blood-brain barrier according to the present invention.

### [Description of the Drawings]

FIG. 1 shows the results of a quantitative analysis with FACS of the penetration degree of brain tumor cells by the sonosensitive liposomes for penetrating the blood-brain barrier according to the present invention, Doxil liposome, and Marquibo liposome.
FIG. 2A shows the results of observing the brain tumor cell infiltration effect of the sonosensitive liposomes for penetrating the blood-brain barrier according to the present invention and Doxil liposome by confocal fluorescence microscopy (red: liposome, blue: nucleus, magnification: 10X).
FIG. 2B shows yet another result of observing the brain tumor cell infiltration effect of the sonosensitive liposomes for penetrating the blood-brain barrier according to the present invention and Doxil liposome by confocal fluorescence microscopy (red: liposome, blue: nucleus, magnification: 10X).
FIG. 3 shows the results of confirming the anticancer cell killing effect depending on the ultrasound treatment of the sonosensitive liposomes for penetrating the blood-brain barrier according to the present invention, Doxil liposome, and free doxorubicin (freeDOX) against brain tumor cells through MTT analysis.
FIG. 4A shows the image of brain tissue infiltration by the blood-brain barrier penetration of Evans-blue (EB) dye depending on the number of microbubbles, and the results of quantitative analysis of the infiltration efficiency.
FIG. 4B shows the results of H&E staining to verify the stability of the brain tissue depending on the number of microbubbles.
FIG. 5A shows the image of brain tissue infiltration by the blood-brain barrier penetration of Evans-blue dye depending on the ultrasound parameters.
FIG. 5B shows the results of quantitative analysis of the infiltration efficiency by the blood-brain barrier penetration of Evans-blue dye depending on the ultrasound parameters.
FIG. 5C shows the results of H&E staining to verify the stability of the brain tissue depending on the ultrasound parameters.
FIG. 6 shows the results of confirming the brain tissue delivery effect and the distribution pattern in main organs by the sonosensitive liposomes for penetrating the blood-brain barrier and Marquibo liposome according to the present invention in a mouse.
FIG. 7 shows the results of confirming the distribution pattern in main organs depending on the ultrasound parameters of liposomes without sphingomyelin (IMP302-004) and liposomes containing sphingomyelin (IMP302-005).
FIG. 8 shows the results of comparing the blood-brain barrier penetration effect of the sonosensitive liposome (IMP302-004) according to the present invention and Doxil liposome.

### [Best Modes of the Invention]

The present invention relates to a composition for penetrating the blood-brain barrier, and the like, and is accomplished by developing sonosensitive liposomes that not only demonstrate superior efficiency in drug encapsulation and ultrasound-induced drug release, but can also effectively penetrate the blood-brain barrier when stimulated by ultrasound.

Specifically, in one embodiment of the present invention, it was confirmed that among the types of aqueous solutions for the liposomes (ammonium sulfate, ammonium citrate, and TEA-SOS), all three liposomes showed a particle size of approximately 200 nm, possessed an excellent drug encapsulation rate, and particularly, loading the drug for 2 hours at 60 °C using ammonium sulfate or ammonium citrate as the aqueous solution proved to be the most optimized condition for drug encapsulation in the liposome (Example 1).

In another embodiment of the present invention, comparison of the physical properties and characteristics of the liposomes depending on the types of lipids composing the liposome revealed that liposomes manufactured with at least one selected from the group consisting of (i) DOPE; and (ii) DSPC, DSPE-mPEG2000, cholesterol, lyso-PC, and sphingomyelin showed the most superior drug encapsulation rate and ultrasound responsiveness (Example 2).

In yet another embodiment of the present invention, when the sonosensitive liposomes according to the invention were co-cultured with a brain tumor cell line, it was confirmed that the liposomes could effectively penetrate into the brain tumor cells (Examples 3 and 4).

In yet another embodiment of the present invention, when the sonosensitive liposomes containing an anticancer drug was co-cultured with a brain tumor cell line while applying ultrasound, it was confirmed that the sonosensitive liposomes exhibited superior anticancer effects on the brain tumor cells (Example 5).

In yet another embodiment of the present invention, to further enhance the drug delivery effect of the sonosensitive liposomes to the brain, parameters for the cavitation of sonication and microbubbles that could stably open the blood-brain barrier were identified (Examples 6 and 7).

In yet another embodiment of the present invention, when the sonosensitive liposomes according to the present invention were administered while opening the blood-brain barrier by applying ultrasound and microbubbles in a mouse model, it was confirmed that the liposomes effectively penetrated into the brain tissue (Examples 8 and 9).

Hence, the sonosensitive liposomes for penetrating the blood-brain barrier according to the present invention have been confirmed to be able to accurately deliver the encapsulated drug to the brain by effectively crossing the blood-brain barrier, and are expected to be utilized as a drug delivery carrier for treating various diseases.

Hereinafter, the present invention will be described in detail.

The present invention provides a composition for penetrating the blood-brain barrier and/or a drug delivery carrier comprising sonosensitive liposomes comprising at least one selected from a group consisting of DSPC (1,2-distearoyl-sn-glycero-3-phosphocholine), DSPE-mPEG2000 (1,2-Distearoyl-sn-glycero-3-phosphoethanolamine-N-[methoxy(polyethylene glycol)-2000]), DOPE (1,2-Dioleoyl-sn-glycero-3-phosphoethanolamine), cholesterol, lyso-PC (lysophosphatidylcholine), and sphingolipid. The composition comprises a pharmaceutical composition.

Preferably, the present invention provides:
i) a composition for penetrating the blood-brain barrier, comprising sonosensitive liposomes as active ingredients, which comprise DSPC (1,2-distearoyl-sn-glycero-3 -phosphocholine), DSPE-mPEG2000 (1,2-Distearoyl- sn-glycero-3 - phosphoethanolamine-N-[methoxy(polyethylene glycol)-2000]), DOPE (1,2-Dioleoyl-sn-glycero-3-phosphoethanolamine), cholesterol, and lyso-PC (lysophosphatidylcholine);
ii) a composition for penetrating the blood-brain barrier, comprising sonosensitive liposomes as active ingredients, which comprises DOPE (1,2-Dioleoyl-sn-glycero-3-phosphoethanolamine) and sphingolipid; and/or
iii) a composition for penetrating the blood-brain barrier, comprising sonosensitive liposomes as active ingredients, which comprises DOPE (1,2-Dioleoyl-sn-glycero-3-phosphoethanolamine), sphingolipid, and DSPE-mPEG2000.

In one embodiment of the present invention, the sonosensitive liposomes can further comprise polysorbate.

In the present invention, "DOPE" is an abbreviation for 1,2-Dioleoyl-sn-glycero-3-phosphoethanolamine, wherein the lipid is known to form heterogeneous liposomes with DOTAP used as a drug delivery vehicle.

In the present invention, "DSPC" is an abbreviation for 1,2-Distearoyl-sn-glycero-3-phosphocholine, referring to the phospholipid composed of two stearic acids attached to the phosphatidylcholine head group.

In the present invention, "DSPE-mPEG2000" is an abbreviation for 1,2-Distearoyl-sn-Glycero-3-Phosphoethanol amine with conjugated methoxyl poly(ethylene glycol2000), referring to the pegylated derivative of 1,2-Distearoyl-sn-Glycero-3-PE (DSPE).

In the present invention, "cholesterol" refers to a lipid found in the cell membranes of all animal cells, one of the sterols (modified steroids). The cholesterol according to the present invention includes derivatives of cholesterol. Derivatives of cholesterol can include, for example, sitosterol, ergosterol, stigmasterol, 4,22-stigmastadien-3-one, stigmasterol acetate, lanosterol, cycloartenol, or a combination thereof. Cholesterol is located in the lipid bilayer, and its amount can be regulated to decrease or increase permeability, and it can be used regardless of the ratio in the liposome.

In the present invention, "Lyso-PC" is an abbreviation for lysophosphatidylcholine, a phospholipid derived from phosphocholine with a head group made up of choline, representative of lipids composed of a single acyl chain, with various types of lyso-PC known.

In the present invention, the lyso-PC may be represented by Chemical Formulas 1 or 2, but is not limited thereto. Preferably, the lyso-PC may be 1-LPC (lysophosphatidylcholine) or 2-LPC represented by Chemical Formulas 1 or 2, but is not limited thereto. 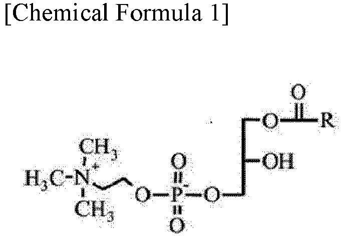 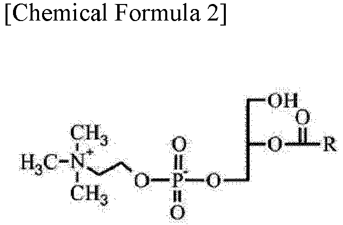 (In the Chemical Formula 1 or 2, R, as referred to herein, may be a C₆ to C₂₆ alkyl, a C₆ to C₂₆ alkenyl, a C₆ to C₂₆ alkynyl, substituted or unsubstituted C₆ to C₂₆ cycloalkyl, substituted or unsubstituted C₆ to C₂₆ aryl, substituted or unsubstituted C₇ to C₂₆ arylalkyl, or H.)

In the present invention, the lyso-PC may be at least one selected from a group consisting of lyso-PC(6:0), lyso-PC(7:0), lyso-PC(8:0), lyso-PC(9:0), lyso-PC(10:0), lyso-PC(11:0), lyso-PC(12:0), lyso-PC(13:0), lyso-PC(14:0), lyso-PC(15:0), lyso-PC(16:0), 2-lyso-PC(16:0), lyso-PC(17:0), lyso-PC(17:1), lyso-PC(18:0), lyso-PC(18:1), lyso-PC(18:2), 2-lyso-PC(18:0), 2-lyso-PC(18:1), lyso-PC(19:0), lyso-PC(20:1), lyso-PC(20:4), lyso-PC(22:0), lyso-PC(22:5), lyso-PC(24:0), lyso-PC(26:0) and lyso-PC(20:5), but is not limited thereto.

Moreover, preferably, the lyso-PC may be MSPC (1-Stearoyl-2-lyso-sn-glycero-3-phosphocholine), but is not limited thereto.

In the present invention, "sphingolipid" refers to lipids containing a skeleton of sphingoid bases to which a fatty acid can attach via an amide bond and a head group of the primary hydroxyl. Sphingolipids are found in all animals, plants, fungi, and some protozoa or viruses, and are known to play important roles in maintaining the structure and function of cell membranes and transmitting signals between cells. In the present invention, the sphingolipid is not limited to any specific type, but preferably, may be at least one selected from a group consisting of sphingosine, ceramide, sphingomyelin, cerebroside, and ganglioside.

Preferably, the sphingolipid according to the present invention is sphingomyelin. In the present invention, "sphingomyelin" refers to a sphingophospholipid composed of phosphocholine and ceramide, or a phosphoethanolamine head group. Natural sphingomyelin is found in the membranes of animal cells, especially in the membranous sheaths surrounding the axons of nerve cells.

In the present invention, "polysorbate" refers to a non-ionic surfactant derived from ethoxylated sorbitan esterified with fatty acids. Polysorbate is commonly known to act as an emulsifying adjuvant and solubilizer in liposomes. In the case of the sonosensitive liposomes according to the present invention, the efficiency of penetrating the blood-brain barrier can be increased by further including polysorbate. In the present invention, polysorbate can be selected from polysorbate 20, polysorbate 40, polysorbate 60, and polysorbate 80, but is not limited thereto. Preferably, the polysorbate referred to herein is polysorbate 80.

In one embodiment of the present invention, the DSPC, DSPE-mPEG2000, DOPE, cholesterol, and lyso-PC may be included in molar ratio (mole%) of 1 to 50, 1 to 10, 5 to 80, 0.1 to 50, and 0.1 to 20, respectively, but is not limited thereto. The inventors have confirmed through specific experiments that liposomes satisfying these composition ratios exhibit excellent drug encapsulation efficiency, ultrasound responsiveness, and blood-brain barrier permeability.

More specifically, the DSPC, DSPE-mPEG2000, DOPE, cholesterol, and lyso-PC are in a molar ratio of 1~50 : 1∼10 : 5~80 : 0.1∼50 : 0.1∼20 mole%, 1∼50 : 1∼10 : 5~68 : 0.1∼50 : 0.1∼20 mole%, 1~50 : 10∼15 : 5~80 : 0.1~50 : 0.1∼20 mole%, 1∼50 : 1~20 : 5~70 : 0.1∼35 : 0.1∼15 mole%, 1∼50 : 1~10 : 5~70 : 0.1∼50 : 0.1∼20 mole%, 1~40 : 1∼9 : 35~80 : 0.1~40 : 1~10 mole%, 1~40 : 1~10 : 20~70 : 0.1∼50 : 1∼10 mole%, 1~40 : 1~10 : 20~68 : 0.1∼50 : 1∼8 mole%, 1~30 : 1~10 : 10~80 : 0.1∼50 : 0.1∼20 mole%, 1~30 : 1∼9 : 5~70 : 0.1~40 : 0.1∼20 mole%, 1~30 : 1∼9 : 5∼68 : 0.1~40 : 0.1∼20 mole%, 1~30 : 1∼10 : 20~80 : 0.1∼50 : 2~10 mole%, 1~30 : 1~20 : 20~68 : 0.1~40 : 1∼11 mole%, 1~20 : 1∼10 : 15~80 : 1~48 : 1~12 mole%, 10∼40 : 3∼7 : 40∼68 : 0.1∼45 : 0.1∼11 mole%, 0.1∼20 : 1∼8 : 63∼67 : 0.1∼32 : 3∼11 mole%, 5∼15 : 1∼7 : 60∼67 : 10∼32 : 1~11 mole%, 8~12 : 3∼6 : 50∼66 : 10∼32 : 5~11 mole%, or 0.1∼20 : 1∼8 : 53∼57 : 0.1-30: 5~10 mole%, but is not limited thereto.

In addition, in the present invention, the DSPC may be included at 1 to 50 mole%, 1 to 40 mole%, 1 to 30 mole%, 1 to 25 mole%, 1 to 20 mole%, 1 to 15 mole%, 1 to 7 mole%, 5 to 50 mole%, 5 to 40 mole%, 5 to 35 mole%, 5 to 30 mole%, 5 to 25 mole%, 5 to 20 mole%, 5 to 15 mole%, 7 to 12 mole%, 25 to 35 mole%, 27 to 32 mole%, or 35 to 45 mole% based on the total liposome, but is not limited thereto.

In addition, in the present invention, the DSPE-mPEG2000 may be included at 1 to 10 mole%, 1 to 9 mole%, 1 to 8 mole%, 1 to 7 mole%, 1 to 6 mole%, 1 to 5 mole%, 2 to 9 mole%, 2 to 8 mole%, 2 to 7 mole%, 2 to 6 mole%, 2 to 5 mole%, 3 to 8 mole%, 3 to 7 mole%, or 4 to 6 mole% based on the total liposome, but is not limited thereto.

Moreover, in the present invention, the DOPE may be included at 5 to 80 mole%, 5 to 75 mole%, 5 to 70 mole%, 5 to 68 mole%, 10 to 75 mole%, 10 to 70 mole%, 10 to 68 mole%, 20 to 75 mole%, 20 to 70 mole%, 20 to 68 mole%, 30 to 75 mole%, 30 to 70 mole%, 30 to 68 mole%, 40 to 80 mole%, 40 to 75 mole%, 40 to 70 mole%, 40 to 68 mole%, 45 to 80 mole%, 45 to 70 mole%, 50 to 80 mole%, 50 to 70 mole%, or 50 to 68 mole% based on the total liposome, but is not limited thereto.

Furthermore, in the present invention, the cholesterol may be included at 0.1 to 50 mole%, 0.1 to 45 mole%, 1 to 45 mole%, 5 to 45 mole%, 5 to 40 mole%, 5 to 35 mole%, 5 to 30 mole%, 5 to 25 mole%, 5 to 20 mole%, 5 to 15 mole%, 10 to 40 mole%, 10 to 35 mole%, 10 to 30 mole%, 10 to 25 mole%, 10 to 20 mole%, 20 to 40 mole%, 20 to 35 mole%, 25 to 40 mole%, 25 to 35 mole%, 27 to 32 mole%, 30 to 40 mole%, 5 to 10 mole%, or 40 to 50 mole% based on the total liposome, but is not limited thereto.

Furthermore, in the present invention, the lyso-PC can be included at 0.1 to 20 mole%, 0.1 to 15 mole%, 0.1 to 10 mole%, 1 to 20 mole%, 1 to 10 mole%, 1 to 9 mole%, 1 to 8 mole%, 1 to 7 mole%, 1 to 6 mole%, 3 to 20 mole%, 3 to 15 mole%, 3 to 10 mole%, 3 to 7 mole%, 2 to 10 mole%, 3 to 7 mole%, 2 to 6 mole%, 5 to 20 mole%, 5 to 15 mole%, 5 to 10 mole%, 7 to 20 mole%, 7 to 15 mole%, 7 to 12 mole%, 8 to 12 mole%, 9 to 11 mole%, 4 to 6 mole%, or 6 to 8 mole% based on the total liposome, but is not limited thereto.

In another embodiment of the present invention, the DSPC can be included in an amount of 0.1 to 50 dry weight% based on the total liposome,
the DSPE-mPEG2000 can be included in an amount of 3 to 50 dry weight% based on the total liposome,
the DOPE can be included in an amount of 1 to 80 dry weight% based on the total liposome,
the cholesterol can be included in an amount of 0.05 to 40 dry weight% based on the total liposome, and
the lyso-PC can be included in an amount of 0.5 to 10 dry weight% based on the total liposome, but it is not limited therto.

In the present invention, the DSPC can be included in an amount of 0.1 to 50 dry weight%, 0.1 to 45 dry weight%, 0.1 to 40 dry weight%, 1 to 40 dry weight%, 1 to 30 dry weight%, 1 to 20 dry weight%, 1 to 15 dry weight%, 1 to 13 dry weight%, 3 to 20 dry weight%, 5 to 20 dry weight%, 7 to 20 dry weight%, 5 to 15 dry weight%, 7 to 13 dry weight%, or 9 to 11 dry weight% based on the total liposome, but it is not limited thereto.

In the present invention, the DSPE-mPEG2000 can be included in an amount of 3 to 50 dry weight%, 5 to 40 dry weight%, 10 to 30 dry weight%, 10 to 25 dry weight%, 10 to 20 dry weight%, 15 to 30 dry weight%, 15 to 25 dry weight%, 15 to 20 dry weight%, 16 to 19 dry weight%, or 17 to 19 dry weight% based on the total liposome, but it is not limited thereto.

In the present invention, the DOPE can be included in an amount of 1 to 80 dry weight%, 5 to 80 dry weight%, 10 to 80 dry weight%, 15 to 80 dry weight%, 20 to 80 dry weight%, 25 to 80 dry weight%, 30 to 80 dry weight%, 30 to 75 dry weight%, 30 to 70 dry weight%, 30 to 65 dry weight%, 30 to 60 dry weight%, 30 to 55 dry weight%, 30 to 50 dry weight%, 40 to 80 dry weight%, 40 to 70 dry weight%, 50 to 80 dry weight%, 50 to 70 dry weight%, 55 to 65 dry weight%, 60 to 65 dry weight%, 60 to 63 dry weight%, or 60 to 62 dry weight% based on the total liposome, but it is not limited thereto.

In the present invention, the cholesterol can be included as 0.05 to 40 dry weight%, 1 to 40 dry weight%, 1 to 30 dry weight%, 1 to 20 dry weight%, 1 to 15 dry weight%, 1 to 13 dry weight%, 1 to 10 dry weight%, 3 to 20 dry weight%, 3 to 15 dry weight%, 3 to 13 dry weight%, 3 to 10 dry weight%, 5 to 20 dry weight%, 5 to 15 dry weight%, 5 to 13 dry weight%, 5 to 10 dry weight%, 6 to 8 dry weight%, 10 to 40 dry weight%, 10 to 35 dry weight%, 10 to 30 dry weight%, 10 to 25 dry weight%, 10 to 20 dry weight%, 15 to 40 dry weight%, 15 to 30 dry weight%, 15 to 25 dry weight%, 15 to 22 dry weight%, or 20 to 30 dry weight% based on the total liposome, but is not limited thereto.

In the present invention, the lyso-PC can be included as 0.5 to 10 dry weight%, 0.5 to 8 dry weight%, 0.5 to 7 dry weight%, 0.5 to 6 dry weight%, 0.5 to 5 dry weight%, 0.5 to 4 dry weight%, 1 to 8 dry weight%, 1 to 7 dry weight%, 1 to 6 dry weight%, 1 to 4 dry weight%, 2 to 8 dry weight%, 2 to 7.5 dry weight%, 2 to 6 dry weight%, 2 to 5 dry weight%, 2 to 4 dry weight%, 2.5 to 7.5 dry weight%, 2.5 to 7 dry weight%, 2.5 to 6.5 dry weight%, 3 to 8 dry weight%, 3 to 7 dry weight%, 3 to 6 dry weight%, 3 to 5 dry weight%, 3 to 4 dry weight%, 5 to 10 dry weight%, 5 to 9 dry weight%, 5 to 8 dry weight%, 5 to 7 dry weight%, 5 to 6 dry weight%, or 5 to 5.5 dry weight% based on the total liposome, but is not limited thereto.

The sonosensitive liposomes according to the present invention comprise DOPE (1,2-Dioleoyl-sn-glycero-3-phosphoethanolamine) and sphingolipid as active ingredients, and may not comprise DSPC, cholesterol, and lyso-PC.

Preferably, the sonosensitive liposomes according to the present invention comprise DOPE, DSPE-mPEG2000, and sphingolipid as active ingredients, and may not comprise DSPC, cholesterol, and lyso-PC.

In one embodiment of the present invention, the DOPE may be included as 5 to 80 mole%, 5 to 75 mole%, 5 to 70 mole%, 5 to 60 mole%, 5 to 50 mole%, 5 to 45 mole%, 5 to 40 mole%, 5 to 35 mole%, 5 to 33 mole%, 5 to 20 mole%, 5 to 10 mole%, 10 to 80 mole%, 10 to 70 mole%, 10 to 60 mole%, 10 to 50 mole%, 20 to 80 mole%, 20 to 70 mole%, 20 to 60 mole%, 20 to 50 mole%, 30 to 80 mole%, 30 to 70 mole%, 30 to 60 mole%, 35 to 55 mole%, or 40 to 55 mole% based on the total liposome, but is not limited thereto.

In another embodiment of the present invention, the sphingolipid may be included as 5 to 80 mole%, 5 to 75 mole%, 5 to 70 mole%, 5 to 60 mole%, 5 to 50 mole%, 5 to 45 mole%, 5 to 40 mole%, 5 to 35 mole%, 5 to 20 mole%, 5 to 10 mole%, 10 to 80 mole%, 10 to 70 mole%, 10 to 60 mole%, 10 to 50 mole%, 20 to 80 mole%, 20 to 70 mole%, 20 to 60 mole%, 20 to 50 mole%, 30 to 80 mole%, 30 to 70 mole%, 30 to 60 mole%, 35 to 55 mole%, or 40 to 55 mole% based on the total liposome, but is not limited thereto.

In yet another embodiment of the present invention, the DSPE-mPEG2000 may be included as 1 to 20 mole%, 1 to 18 mole%, 1 to 16 mole%, 1 to 14 mole%, 1 to 12 mole%, 5 to 20 mole%, 6 to 20 mole%, 7 to 20 mole%, 8 to 20 mole%, 9 to 20 mole%, 7 to 15 mole%, 7 to 13 mole%, 5 to 15 mole%, 5 to 13 mole%, or 8 to 12 mole%, but is not limited thereto.

In another embodiment of the present invention, the DOPE, sphingolipid, and DSPE-mPEG2000 may be included in mole% of 20~60 : 20~60 : 1~20, 20~70 : 20~70 : 5∼15, 30∼60 : 20~70 : 5~20, 35∼50 : 35~50 : 8~15, 25~60 : 25∼60 : 8∼15, or 40∼55 : 40~55 : 8∼15, but is not limited thereto.

Furthermore, in the present invention, the DOPE may be included as 1 to 80 dry weight% based on the total liposome, the sphingolipid may be included as 1 to 80 dry weight% based on the total liposome, the DSPE-mPEG2000 may not be included or may be included as 3 to 50 dry weight% based on the total liposome, but is not limited thereto.

The liposome of the present invention is formed by amphipathic compounds containing phospholipids. Such amphipathic compounds are typically arranged at the interface between an aqueous medium and an essentially insoluble organic solvent, stabilizing emulsified solvent microdroplets. The amphipathic compound includes a molecule having a hydrophilic polar head portion (for example, polar or ionic group) that can react with an aqueous medium, and a hydrophobic organic tail portion (for example, hydrocarbon chain) that can react with, for example, an organic solvent. Amphipathic compounds are compounds that can stabilize mixtures of substances that cannot usually be mixed by other methods, such as mixtures of two immiscible liquids (for example, water and oil), mixtures of liquids and gases (for example, gas microbubbles in water), or mixtures of liquids and insoluble particles (for example, metal nanoparticles in water).

Furthermore, the sonosensitive liposomes according to the present invention may be hydrated with ammonium sulfate, ammonium citrate, or TEA-SOS. In other words, the inner buffer of the liposome may be ammonium sulfate, ammonium citrate, or TEA-SOS. Preferably, the sonosensitive liposomes according to the present invention are hydrated with ammonium sulfate or ammonium citrate.

The sonosensitive liposomes according to the present invention can penetrate the blood-brain barrier (i.e., pass through or move). In other words, the sonosensitive liposomes can penetrate the blood-brain barrier and move into the brain tissue. Particularly, the sonosensitive liposomes according to the present invention can penetrate the blood-brain barrier while maintaining its physical properties and characteristics. Therefore, the sonosensitive liposomes according to the present invention can be used as a carrier for delivering drugs into the brain, i.e., into the brain tissue. That is, the composition for penetrating the blood-brain barrier according to the present invention may be for delivering a drug encapsulated in the sonosensitive liposomes into the brain tissue.

In the present invention, the "Blood-brain barrier (BBB)" refers to a vascular barrier that isolates the brain and blood, blocking foreign substances such as pigments, drugs, and toxins from entering the brain tissue, thereby protecting the brain. The blood-brain barrier is distributed overall in the cerebral blood vessels surrounding the brain cells, where the cerebral capillary endothelial cells are tightly combined with the blood-brain barrier, and the surrounding glial cells tightly envelop it, preventing drugs or metabolites from entering. The substances that constitute the blood-brain barrier are mostly composed of phospholipids.

The liposome according to the present invention passes through the blood-brain barrier when exposed to ultrasound.

The term "ultrasound" used in the present invention generally refers to sound waves exceeding the frequency of audible sound, which is 16 Hz to 20 kHz, that human ears can hear. High-intensity focused ultrasound introduces a focused type of ultrasound that provides continuous and high-intensity ultrasound energy to a focal point, which can produce instantaneous thermal effects (65-100°C), cavitation effects, mechanical effects, and sonochemical effects depending on the energy and frequency. While ultrasound is not harmful when passing through human tissue, high-intensity ultrasound that forms a focus generates sufficient energy to cause coagulative necrosis and thermal ablation effects, regardless of the type of tissue.

In the present invention, the liposomes are sonosensitive liposomes. Sonosensitive liposomes may mean liposomes whose permeability increases when exposed to ultrasound. Therefore, when liposomes are exposed to ultrasound, the drug loaded in the liposomes can be released. Alternatively, sonosensitive liposomes may mean liposomes whose blood-brain barrier penetration ability increases when exposed to ultrasound. Therefore, when liposomes are exposed to ultrasound, the liposomes can pass through the blood-brain barrier and be delivered to the brain, and likewise, the drug that was encapsulated in the liposomes can be delivered to the brain by ultrasound.

In the present invention, the ultrasound refers to sound waves with a frequency greater than the audible frequency range of 16 Hz to 20 kHz. The ultrasound can be high intensity focused ultrasound (HIFU), high intensity unfocused ultrasound, or a combination of both, but is not limited thereto. HIFU refers to an ultrasound that concentrates high intensity ultrasound energy to create a focused point. Depending on which image is viewed during the high-intensity focused ultrasound treatment, there are Ultrasound-guided HIFU and Magnetic Resonance Imaging-guided HIFU. The frequency of the ultrasound can be, for example, 1 kHz to 100 kHz, 1 kHz to 90 kHz, 1 kHz to 80 kHz, 1 kHz to 70 kHz, 1 kHz to 60 kHz, 1 kHz to 50 kHz, 1 kHz to 40 kHz, 1 kHz to 30 kHz, 1 kHz to 20 kHz, 1 kHz to 10 kHz, 20 kHz to 3.0 MHz, 40 kHz to 2.0 MHz, 60 kHz to 2.0 MHz, 80 kHz to 2.0 MHz, 100 kHz to 2.0 MHz, 150 kHz to 2.0 MHz, 200 kHz to 2.0 MHz, 250 kHz to 2.0 MHz, 300 kHz to 2.0 MHz, 350 kHz to 2.0 MHz, 400 kHz to 2.0 MHz, 450 kHz to 2.0 MHz, 500 kHz to 2.0 MHz, 550 kHz to 2.0 MHz, 600 kHz to 2.0 MHz, 650 kHz to 2.0 MHz, 700 kHz to 2.0 MHz, 750 kHz to 2.0 MHz, 800 kHz to 2.0 MHz, 850 kHz to 2.0 MHz, 900 kHz to 2.0 MHz, 950 kHz to 2.0 MHz, 600 kHz to 1.5 MHz, 650 kHz to 1.5 MHz, 700 kHz to 1.5 MHz, 750 kHz to 1.5 MHz, 800 kHz to 1.5 MHz, 850 kHz to 1.5 MHz, 900 kHz to 1.5 MHz, 950 kHz to 1.5 MHz, 1 MHz to 1.5 MHz, 600 kHz to 1.3 MHz, 650 kHz to 1.3 MHz, 700 kHz to 1.3 MHz, 750 kHz to 1.3 MHz, 800 kHz to 1.3 MHz, 850 kHz to 1.3 MHz, 900 kHz to 1.3 MHz, 950 kHz to 1.3 MHz, 600 kHz to 1.1 MHz, 650 kHz to 1.1 MHz, 700 kHz to 1.1 MHz, 750 kHz to 1.1 MHz, 800 kHz to 1.1 MHz, 850 kHz to 1.1 MHz, 900 kHz to 1.1 MHz, 950 kHz to 1.1 MHz, 600 kHz to 1 MHz, 650 kHz to 1 MHz, 700 kHz to 1 MHz, 750 kHz to 1 MHz, 800 kHz to 1 MHz, 850 kHz to 1 MHz, 900 kHz to 1 MHz, or 950 kHz to 1 MHz, but is not limited thereto.

The liposome may be ensured of stability. The stability refers to a condition where the drug encapsulated in the liposome in the bloodstream does not release when not exposed to ultrasound. The inventors of the present invention have confirmed that when the release rate of the drug encapsulated in the sonosensitive liposomes according to the present invention was measured every 20 minutes for 60 minutes under vortexing conditions at room temperature (20~25°C), it showed a maximum drug release rate of 30% or less. In other words, the liposome according to the present invention maintains a stable structure in the absence of ultrasound, and therefore, the drug is hardly released.

The particle size of the liposome according to the present invention can be, for example, 50 nm to 500 nm, 50 nm to 400 nm, 50 nm to 300 nm, 50 nm to 200 nm, 60 nm to 200 nm, 70 nm to 200 nm, 80 nm to 200 nm, 90 nm to 200 nm, 100 nm to 200 nm, 110 nm to 190 nm, 120 nm to 180 nm, 130 nm to 170 nm, 140 nm to 170 nm, 140 nm to 160 nm, or about 150 nm. According to a specific embodiment, it can be 100 nm to 200 nm. The particle size distribution of the liposomes can be measured using a Zetasizer Nano ZS (Malvern) by the Dynamic Light Scattering (DLS) analysis method after diluting the liposomes 10 fold. However, the method of measuring the particle size distribution of liposomes is not limited thereto, and can be measured according to other methods disclosed in the art, and can be converted to equivalent values.

The sonosensitive liposomes according to the present invention can encapsulate a drug. The term "encapsulation" can be interchangeably used with the terms "incorporation" or "loading".

The term "drug" as used in the present invention refers to any compound with a desired biological activity. The desired biological activity includes activity useful for diagnosing, healing, alleviating, treating, or preventing disease in humans or other animals.

In the present invention, the mixing ratio of the drug and the sonosensitive liposomes can preferably be 1:2 to 1:50 mass ratio (w/w%), more preferably 1:2 to 1:40, 1:2 to 1:35, 1:2 to 1:30, 1:2 to 1:25, 1:10 to 1:50, 1:10 to 1:40, 1:10 to 1:35, 1:10 to 1:30, 1:10 to 1:35, 1:2 to 1:20, 1:2 to 1:18, 1:2 to 1:16, 1:2 to 1:14, 1:2 to 1:12, 1:2 to 1:10.5, 1:2.5 to 1:10.5, 1:2.5 to 1:5, 1:2 to 1:4, 1:2.5 to 1:4.5, 1:2.5 to 1:4, 1:2.5 to 1:3, 1:1:5 to 1:10.5, 1:5.5 to 1:10.5, 1:6 to 1:10.5, 1:6.5 to 1:10.5, 1:7 to 1:10.5, 1:7 to 1:10, 1:7 to 1:9, or about 1:8 mass ratio (w/w%), but is not limited thereto, as long as the drug can be efficiently incorporated into the liposome.

When a drug is treated with the liposome to encapsulate it into the liposome according to the present invention, the ratio of the drug encapsulated in the liposome to the total drug added can be 50 to 100%. That is, the liposome according to the present invention can have a drug encapsulation rate of 30% to 100%, 40% to 100%, 50% to 100%, 60% to 100%, 65% to 100%, 70% to 100%, 75% to 100%, 76% to 100%, 77% to 100%, 80% to 100%, 85% to 100%, 87% to 100%, 88% to 100%, 90% to 100%, or 95% to 100%, but is not limited thereto.

The encapsulation rate can mean the loading rate for the amount of drug added, but is not limited thereto. The method of measuring the drug encapsulation rate in the liposome involves treating the drug with the liposome, separating the unencapsulated drug using size exclusive chromatography (SEC), and calculating by measuring the absorbance of the encapsulated drug and the unencapsulated drug. However, the method of measuring the drug encapsulation rate is not limited to this, and it can be measured according to other methods disclosed in the art, and can be converted to an equivalent level of value.

In the present invention, the drug-encapsulated sonosensitive liposomes can have a drug release rate of 10% to 100%, 50% to 100%, 60% to 100%, 65% to 100%, 70% to 100%, or 75% to 100%, but is not limited thereto. The drug release rate can mean the release rate for the amount of drug added, but is not limited thereto. Here, the method of measuring the release rate involves treating the liposome with ultrasound, separating the drug released from the liposome and the liposome from which the drug was released using the SEC method, and quantifying by measuring the absorbance of the drug. However, the method of calculating the drug release rate is not limited thereto, and it can be measured according to other methods disclosed in the art, and can be converted to an equivalent level of value.

In the present invention, the drug-encapsulated sonosensitive liposomes can have a drug release rate of 0.1% to 100%, 0.1% to 80%, 0.1% to 60%, 0.1% to 40%, 0.1% to 30%, 0.1% to 20%, 0.1% to 10%, or less than 30% in the bloodstream under ultrasound untreated conditions, but is not limited thereto.

The composition according to the present invention can be used for the purpose of delivering a drug through the blood-brain barrier to the brain. That is, the present invention provides a blood-brain barrier penetrating drug delivery carrier comprising the sonosensitive liposomes as active ingredients according to the present invention.

In the present invention, the "drug" can include without limitation any drug that acts in the brain. That is, the drug can include without limitation any drug intended for delivery to the brain, regardless of its specific type or components. Preferably, the drug can be a therapeutic agent for brain disease.

In the present invention, "brain disease" refers to a pathological state in which the function or structure of brain tissue is reversibly or irreversibly reduced or lost due to damage to the brain tissue, blood vessels, and nerves. Preferably, the brain disease can be selected from a group consisting of brain tumors, brain infections caused by bacteria or viruses, Parkinson's disease, encephalitis, stroke, palsy, Alzheimer's, Lou Gehrig's disease, Huntington's disease, Pick's disease, Creutzfeldt-Jakob disease, epilepsy, thrombosis, embolism, brain infarction, palsy, small artery occlusion, and cerebral metabolic disorders, but is not limited thereto, and includes all diseases related to the brain.

Most preferably, the brain disease can be a brain tumor (brain cancer). Preferably, the brain tumor can be selected from a group consisting of astrocytoma, glioma, brainstem glioma, pituitary adenoma, glioblastoma, oligodendroglioma, glioblastoma multiforme, oligodendroglioma, oligoastrocytoma, ependymoma, medulloblastoma, hemangioblastoma, meningioma, pituitary adenoma, craniopharyngioma, and choroid plexus papilloma, but is not limited thereto.

The terms "cancer" or "tumor" used herein refer to a disease caused by cells that ignore the normal limits of growth and exhibit aggressive characteristics of division and growth, invasive characteristics of invading surrounding tissues, and metastatic characteristics of spreading to other parts of the body.

The term "therapeutic agent for brain disease" may include at least one selected from the group consisting of vincristine, vinblastine, vinflunine, vindesine, vinorelbine, temozolomide, carmustine, lomustine, cabazitaxel, docetaxel, larotaxel, ortataxel, paclitaxel, tesetaxel, ixabepilone, lomustine, procarbazine, rituximab, tocilizumab, temozolomide, carboplatin, erlotinib, irinotecan, enzastaurin, vorinostat, doxorubicin, cisplatin, Gleevec, 5-fluorouracil, tamoxifen, topotecan, belotecan, imatinib, floxuridine, gemcitabine, leuprolide, flutamide, zoledronate, methotrexate, camptothecin, hydroxyurea, streptozocin, valrubicin, retinoic acid, mechlorethamine, chlorambucil, busulfan, doxifluridine, mitomycin, prednisone, Afinitor, mitoxantrone, levodopa, carbidopa, entacapone, tolcapone, dopamine agonists, donepezil, galantamine, rivastigmine, memantine, anticholinergics, and amantadine, but is not limited thereto.

In addition, the present invention provides a pharmaceutical composition for preventing or treating brain disease, which comprises the sonosensitive liposomes according to the present invention as active ingredients. Preferably, the sonosensitive liposomes encapsulate a therapeutic agent for brain disease.

In the present invention, "preventing" refers to all actions of inhibiting or delaying the onset of brain disease by administering the composition according to the present invention.

In the present invention, "treating" refers to all actions in which the symptoms of brain diseases are improved or favorably altered by the administration of the composition according to the present invention.

The term "pharmaceutical composition" used herein refers to something manufactured for the purpose of preventing or treating brain disease, and can be formulated in various forms according to conventional methods. For example, it can be formulated into oral forms such as powders, granules, tablets, capsules, suspensions, emulsions, and syrups, or into forms for external use on the skin such as creams, gels, patches, sprays, ointments, patches, lotions, liniments, pastes, or cataplasmas, or into forms such as suppositories and sterile injection solutions.

The composition may further comprise at least one selected from a group consisting of anticancer agents, imaging contrast agents, antibiotics, antiinflammatory agents, proteins, cytokines, peptides, and antibodies, in addition to the sonosensitive liposomes according to the present invention.

In addition, the pharmaceutical composition according to the present invention may further comprise appropriate carrier, excipient, and diluent conventionally used in the manufacture of pharmaceutical compositions. As the carrier, the excipient, and the diluent that may be included in the pharmaceutical composition according to the present invention, lactose, dextrose, sucrose, oligosaccharides, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil may be used. For formulation, commonly used diluents or excipients such as fillers, thickeners, binders, wetting agents, disintegrants, and surfactants are used. Solid preparations for oral administration include tablets, pills, powders, granules, capsules, and the like, and such solid preparations are formulated by mixing the composition with at least one excipient, e.g., starch, calcium carbonate, sucrose, lactose, gelatin, and the like. In addition to simple excipients, lubricants such as magnesium stearate and talc are also used. Examples of liquid preparations for oral administration include suspensions, liquids for internal use, emulsions, syrups, and the like, and these liquid preparations may include, in addition to simple commonly used diluents, such as water and liquid paraffin, various types of excipients, for example, a wetting agent, a sweetener, a fragrance, a preservative, and the like. Preparations for parenteral administration include an aqueous sterile solution, a non-aqueous solvent, a suspension, an emulsion, a freeze-dried preparation, and a suppository. Nonlimiting examples of the non-aqueous solvent and the suspension include propylene glycol, polyethylene glycol, a vegetable oil such as olive oil, and an injectable ester such as ethyl oleate. For the base of suppositories, substances such as witepsol, macrogol, tween 61, cocoa butter, laurin butter, and glycerogelatin can be used.

The pharmaceutical composition of the present invention may be administered to a subject via various routes. All administration methods can be predicted, and the pharmaceutical composition may be administered via, for example, oral administration, subcutaneous injection, intraperitoneal injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration, or the like.

The pharmaceutical composition according to the present invention is administered in a pharmaceutically effective amount. In the present invention, the "pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, and simultaneously used drugs, and factors well known in other medical fields.

The composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art. Administration can be done once a day, or it can be divided into several doses throughout the day.

As used herein, the "administration" refers to providing a subject with a predetermined composition of the present invention by using an arbitrary appropriate method.

The "subject" used herein refers to a subject in need of treatment of a disease, and more specifically, mammals such as a human or non-human primates, mice, dogs, cats, horses, and cattle.

The composition according to the present invention can be administered sequentially or simultaneously with ultrasound treatment. Preferably, the composition can be administered immediately after the ultrasound treatment. In particular, the ultrasound can be applied to the brain. The inventors of the present invention have confirmed safe ultrasound parameters that can open the blood-brain barrier without causing damage to brain tissue, and when administering the pharmaceutical composition while emitting ultrasound under these conditions, the efficiency of drug delivery to the brain by the liposomes according to the present invention can be further enhanced.

Preferably, the frequency of the ultrasound can be 20 kHz to 3 MHz, 20 kHz to 2 MHz, 20 kHz to 1.5 MHz, or 20 kHz to 1 MHz, but is not limited thereto.

Preferably, the intensity of the ultrasound can be 0.1 to 5 W, 0.1 to 4 W, 0.1 to 3 W, 0.1 to 2 W, 0.1 to 1.5 W, 0.5 to 3 W, 0.5 to 2 W, or 0.7 to 1.5 W, but is not limited thereto.

Moreover, the duty cycles of the ultrasound can be 0.5 to 20%, 0.5 to 15%, 0.5 to 12%, 0.5 to 10%, 0.5 to 7%, 0.7 to 10%, 0.8 to 10%, 0.9 to 10%, 1 to 10%, 1 to 9%, 1 to 8%, 1 to 7%, 1 to 6%, 1 to 5%, 1 to 4%, 1 to 3%, 2 to 10%, 3 to 10%, or 4 to 10%, but is not limited thereto.

Moreover, the ultrasound may be emitted for 10 to 300 seconds, 10 to 250 seconds, 10 to 200 seconds, 10 to 150 seconds, 10 to 120 seconds, 10 to 100 seconds, 10 to 90 seconds, 10 to 80 seconds, 10 to 70 seconds, 20 to 100 seconds, 30 to 100 seconds, 40 to 100 seconds, 50 to 100 seconds, 50 to 90 seconds, 50 to 80 seconds, or 50 to 70 seconds, but is not limited thereto.

Furthermore, the ultrasound treatment can be carried out sequentially or simultaneously with the administration of microbubbles. Preferably, the ultrasound treatment can be performed immediately after the administration of microbubbles. In the present invention, microbubbles refer to bubbles of an average size of 1 to 10 µm that act as ultrasound contrast agents, causing cavitation in conjunction with ultrasound and temporarily opening the blood-brain barrier.

Preferably, the microbubbles may be administered at 10 to 1×10¹⁰ particles/g, 10 to 1×10⁹ particles/g, 10 to 1×10⁸ particles/g, 10 to 1×10⁷ particles/g, 10 to 1×10⁶ particles/g, 10 to 1×10⁵ particles/g, 10×10² to 1×10⁸ particles/g, 10×10³ to 1×10⁸ particles/g, 10×10⁴ to 1×10⁸ particles/g, 1×10⁶ to 9×10⁶ particles/g, 1×10⁶ to 8×10⁶ particles/g, 1×10⁶ to 7×10⁶ particles/g, 1×10⁶ to 6×10⁶ particles/g, 2×10⁶ to 7×10⁶ particles/g, or 4×10⁶ to 7×10⁶ particles/g based on the total weight of the subject, but is not limited thereto.

Furthermore, the present invention provides a method for producing the sonosensitive liposomes, comprising the following steps:
(S1) dissolving at least one selected from the group consisting of DSPC, DSPE-mPEG2000, DOPE, cholesterol, and lyso-PC in a first organic solvent;
(S2) evaporating the organic solvent to manufacture a liposome film; and
(S3) hydrating the liposome film with an aqueous solution.

In the present invention, the first organic solvent may be at least one selected from the group consisting of dimethylacetamide, dimethylformamide, dimethyl sulfoxide, chloroform, methanol, ethanol, and ether, but is not limited thereto.

In the present invention, the aqueous solution may be ammonium sulfate, ammonium citrate, or TEA-SOS, but is not limited thereto, and may be appropriately selected depending on the type of drug to be encapsulated in the liposome. Most preferably, the aqueous solution is ammonium sulfate.

In the present invention, the first organic solvent in the (S1) step may contain polysorbate, but is not limited thereto.

The manufacturing method may further include the step of extruding the liposome hydrated after the (S3) step through an extruder. The temperature for the extrusion of the liposome can be variously adjusted from room temperature to the transition temperature range of each material, and the number of extrusions can be repeated a suitable number of times to make the size of the liposome uniform.

Furthermore, the present invention provides a method for encapsulating a therapeutic agent for brain disease in the sonosensitive liposomes for penetrating the blood-brain barrier, which includes the step of mixing a brain disease therapeutic and the sonosensitive liposomes.

Preferably, the mixing step may be carried out at 25 to 70 °C, 30 to 70 °C, 40 to 70 °C, 50 to 70 °C, 55 to 70 °C, 50 to 65 °C, or 55 to 65 °C.

Moreover, the mixing step may be carried out for 30 minutes to 5 hours, 30 minutes to 4 hours, 30 minutes to 3 hours, 1 hour to 5 hours, 1 hour to 4 hours, or 1 hour to 3 hours.

The terms used herein have been chosen from generally used terms currently widely used, considering their functions in the present invention. However, these may vary according to the intention of the technicians working in the field, precedents, the emergence of new technologies, etc. In addition, in certain cases, there are terms arbitrarily selected by the applicant, and in this case, the meaning of the terms will be detailed in the description section of the corresponding invention. Therefore, the terms used herein should not be defined solely by the name of the term, but should be defined based on the meaning of the term and the overall content of the present invention.

Throughout the specification of the present invention, when a portion is said to "comprise" a certain component, unless otherwise specifically indicated, it does not exclude other components but may further comprise other components. Terms of degree such as "about", "substantially" used throughout the specification of the present invention are used in a meaning close to or at the number when manufacturing and material tolerances inherent to the mentioned meaning are presented, and are used to prevent unscrupulous infringers from unfairly exploiting the disclosure where exact or absolute numerical values are mentioned to aid the understanding of the present invention.

Throughout the specification of the present invention, the term "combinations thereof" included in the Markush-type expressions refers to a mixture or combination of at least one selected from the group consisting of the components listed in the Markush-type expressions, which means including at least one selected from the group consisting of these components.

Hereinafter, preferred examples are provided to assist the understanding of the present invention. However, the following examples are provided only to facilitate understanding of the present invention, and the content of the present invention is not limited by the following examples.

### [Examples]

The materials used in the manufacture of the sonosensitive liposomes for penetrating the blood-brain barrier according to the present invention are as listed in the following table.

| Phospholipids | IUPAC |
|---|---|
| DSPC | 1,2-Distearoyl-sn-glycero-3-phosphocholine |
| DSPE-mPEG2000 | 1,2-Distearoyl-sn-Glycero-3-Phosphoethanol amine with conjugated methoxy poly(ethylene glycol2000) |
| DOPE | 1,2-Dioleoyl-sn-glycero-3-phosphoethanolamine |
| Cholesterol | Cholesterol |
| lyso-PC | 1-oleoyl-2-hydroxy-sn-glycero-3-phophocholine |
| Sphingomyelin | N-hexadecanoyl-D-erythro-sphingosylphosphorylcholine |

### Example 1. Manufacture of Liposomes and Encapsulation of Drugs for Penetrating the Blood-brain Barrier and Drug Delivery

Sonosensitive liposomes (IMP302), optimized for the treatment of brain tumors, were manufactured. Initially, to evaluate the solvent's impact on drug loading efficiency, liposomes with the lipid composition shown in Table 1 were manufactured and experimented with.

**[Table 1]**

| **Components and Composition Ratios (molar ratio, %) of Liposomes for Penetrating the Blood-brain Barrier and Drug Delivery** | | | | | |
|---|---|---|---|---|---|
| | **DSPC** | **DSPE-PEG** | **Cholesterol** | **DOPE** | **MSPC** |
| **Molar ratio (%)** | 10 | 5 | 30 | 65 | 5 |
| **Amount (mg)** | 2.99 | 5.31 | 4.39 | 18.3 | 0.98 |

### 1-1. Manufacturing and Hydration of Lipid Film

The total amount of lipids was set to 32 mg for each sample, and all were dissolved in 2 ml of chloroform. A thin film of lipid was produced by completely evaporating the chloroform using a rotary evaporator. Thereafter, 250 mM of ammonium sulfate, ammonium citrate, or TEA-SOS was used as an interior buffer to hydrate each lipid film. Hydration occurred by stirring the lipid film and each solution at 50 °C at 200 rpm, hydrating at a lipid concentration of 32 mg/mL.

### 1-2. Size Control of Liposomes Using Size Extrusion

The liposomes produced through 1-1 have a multi-lamellar structure, and the size distribution is also polydisperse. To transform the liposomes into a unilamellar structure and control the size to 100 to 200 nm, a size extruder mini (Avanti) equipped with a polycarbonate filter was used. Size extrusion was controlled using a polycarbonate filter with a pore size of 200 nm. Ten to twenty reciprocal extrusions were performed with a syringe to obtain monodisperse liposomes.

### 1-3. Exchange of Exterior Buffer

To induce remote loading, the exterior buffer of the manufactured liposomes was replaced with deionized water (DW) using a PD-10 column. The exchange was carried out by loading 2 mL of liposome solution into a PD-10 column and then eluting with 4 mL of DW, thereby obtaining liposomes with the exterior buffer replaced with deionized water.

### 1-4. Encapsulation of Vincristine Sulfate

To encapsulate vincristine sulfate into the obtained liposomes, vincristine sulfate and lipids were mixed in a ratio of 1:20 (w/w %) in deionized water, then stirred at 37 °C or 60 °C at 150 rpm for 2 hours. Subsequently, non-encapsulated vincristine sulfate was removed using a PD-10 column in the same method described above. The loading efficiency of vincristine in the liposomes was quantitatively analyzed by measuring the absorbance at 294 nm using UV-vis.

### 1-5. Verification of Characteristics and Optimization of Manufacturing Method of Sonosensitive Liposomes for penetrating the blood-brain barrier

Sonosensitive liposomes for penetrating the blood-brain barrier, manufactured via the lipid thin film hydration method according to the example above, were obtained. To verify the characteristics of the liposomes depending on the type of hydration solution used for liposome manufacturing, the size distribution of the liposomes and the encapsulation efficiency of vincristine sulfate were compared according to the type of hydration solution. The results are shown in Table 2 below.

**[Table 2]**

| **Results of Size Distribution and Vincristine Sulfate Encapsulation Efficiency Depending on the Interior Buffer Used in Manufacturing Sonosensitive Liposomes for Penetrating the Blood-brain Barrier** | | |
|---|---|---|
| **Interior buffer** | **Size distribution (d.nm)** | **Loading efficiency (w/w %, 60°C)** |
| Ammonium sulfate (250mM) | 159.1 ± 69.2 | 4.6 |
| Ammonium citrate (250mM) | 140.2 ± 38.9 | 4.9 |
| TEA-SOS (250mM) | 220.3 ± 75.2 | 4.2 |

As can be seen in Table 2, the size distribution of the sonosensitive liposomes for penetrating the blood-brain barrier, manufactured using each internal buffer, all showed a size of approximately 200nm. Also, the encapsulation efficiency (w/w %) of vincristine sulfate for each liposome was shown to be at a level corresponding to the encapsulation efficiency for the commercial liposome formulation, Marqibo.

Moreover, to determine the optimal conditions for loading vincristine sulfate into the sonosensitive liposomes for penetrating the blood-brain barrier manufactured with ammonium citrate, a comparative evaluation was conducted using UV-vis to measure loading efficiency based on temperature and time during the mixing stage of vincristine sulfate and the liposomes. The loading effects for each temperature or time condition are as follows.

**[Table 3]**

| **Comparative Evaluation of Vincristine Sulfate Loading Efficiency into Sonosensitive Liposomes for Penetrating the Blood-brain Barrier Based on Time and Temperature** | | |
|---|---|---|
| **Experimental Condition** | **Size distribution (d.nm)** | **Loading efficiency (w/w%)** |
| Temperature (37 °C, 2h) | 128.7 ± 40.1 | 3.5 |
| Temperature (60 °C, 2h) | 132.5 ± 35.9 | 4.7 |
| Loading time (60 °C, 24h) | 135.5 ± 42.4 | 5.1 |

Loading efficiency of vincristine sulfate varied with temperature, showing 3.5% at 37°C and 4.7% at 60°C. Thus, it was demonstrated to be more effective in terms of loading efficiency to load vincristine sulfate at a temperature of 60°C. Also, when comparing the loading time conditions of 2 hours and 24 hours, both showed similar loading efficiency. It was found that the size distribution of the liposomes remained stable without changes during the loading process of vincristine sulfate.

Through the example, it was finally confirmed that the sonosensitive liposomes for penetrating the blood-brain barrier, loaded with vincristine sulfate, was most effectively manufactured with either ammonium citrate (pH 3.2) or ammonium sulfate (pH 6.4) as the internal buffer. For more effective loading, it was found that the optimal conditions involved loading the therapeutic onto the liposome at 60°C for 2 hours.

### Example 2. Comparison of the Characteristics of Sonosensitive Liposomes for Penetrating the Blood-brain Barrier Depending on Lipid Composition

To identify the optimal composition of sonosensitive liposomes for treating brain tumors, we compared the physical properties and characteristics depending on the type of lipid composing the liposomes. The compositions of each liposome manufactured for comparison experiments are as shown in Table 4 below.

**[Table 4]**

| **Composition and Molar ratio (%) of Constituents of Sonosensitive Liposomes for Penetrating the Blood-brain Barrier** | | | | | | | |
|---|---|---|---|---|---|---|---|
| **Lipid Composition No.** | **Lipid composition and molar ratio (%)** | | | | | | |
| | **DSPC** | **DSPE-mPEG2k** | **cholesterol** | **DOPE** | **MSPC** | **sphingomyelin** | **Polysorbate 80** |
| 004 | 10 | 5 | 30 | 65 | 5 | - | - |
| 004P | 10 | 5 | 30 | 65 | 5 | | Addition |
| 005 | - | 10 | - | 45 | - | 45 | - |
| 006 | - | - | - | 50 | - | 50 | - |
| 007 | - | - | - | 10 | - | 90 | - |
| 008 | - | - | - | 90 | - | 10 | - |
| 009 | - | - | 10 | 45 | - | 45 | - |
| 010 | - | - | - | 60 | - | 40 | - |
| 011 | - | - | - | 30 | - | 70 | - |
| 012 | - | - | - | 20 | - | 80 | - |
| 013 | - | - | - | 70 | - | 30 | - |
| 013P | | | | 69.95 | | 29.95 | Addition |
| *Polysorbate 80 was added to the solvent at 0.1%(v/v) for preparation. | | | | | | | |

Each liposome was manufactured according to the method of Example 1, but doxorubicin-HCl, which has a higher loading efficiency than vincristine, was used for clear comparison. Formulations 004P and 013P containing Polysorbate 80 were prepared by adding 0.1% (v/v) of Polysorbate 80 to the solvent.

### 2-1. Encapsulation and Encapsulation Efficiency Analysis of Doxorubicin-HCl

The encapsulation of doxorubicin-HCl was achieved by mixing doxorubicin-HCl and liposomes at a weight ratio of 1:8, followed by stirring at 150 rpm for 2 hours at 37°C. Unloaded doxorubicin-HCl was removed using a PD-10 column. The doxorubicin-HCl loaded into the liposomes was quantified by analyzing the absorbance at 475 nm using UV-vis.

Through basic physical property analysis, primary selection of candidate compositions for sonosensitive liposomes for treating brain tumor was conducted, and the optimal composition was derived through drug release tests by ultrasound. The effects of component size distribution and doxorubicin-HCl encapsulation in the sonosensitive liposomes for penetrating the blood-brain barrier are shown in Table 5 below.

**[Table 5]**

| **Liposome Composition by Component Ratio; and Comparison of the Physical Properties and Doxorubicin-HCl Loading Efficiency of Each Liposome** | | | | | | | | | | | | | |
|---|---|---|---|---|---|---|---|---|---|---|---|---|---|
| **Lipid** | **Composition No.** | | | | | | | | | | | | |
| | 004 | 004P | 005 | 006 | 007 | 008 | 009 | 010 | 011 | 012 | 013 | 013P | Marq ibo |
| DOPE | 65 | 65 | 45 | 50 | 10 | 90 | 45 | 40 | 30 | 20 | 70 | 70 | |
| sphingo myelin | | | 45 | 50 | 90 | 10 | 45 | 60 | 70 | 80 | 30 | 30 | 60 |
| DSPE-mPEG2 k | 5 | 5 | 10 | | | | | | | | | | |
| Choleste rol | 30 | 30 | | | | | 10 | | | | | | 40 |
| DSPC | 10 | 10 | | | | | | | | | | | |
| MSPC | 5 | 5 | | | | | | | | | | | |
| Polysorb ate 80 | | 0.1% (v/v) | | | | | | | | | | 0.1% (v/v) | |

| **Experimental Results** | | | | | | | | | | | | | |
|---|---|---|---|---|---|---|---|---|---|---|---|---|---|
| Size (d.nm) | 123.9 | 128.2 | 135.2 | 143.6 | 127.8 | X (Unst able partic les) | 128.2 | 139.1 | 134.2 | 122.5 | 123.5 | 146.2 | 141.3 |
| PDI | 0.062 | 0.075 | 0.055 | 0.079 | 0.089 | | 0.092 | 0.096 | 0.112 | 0.091 | 0.095 | 0.097 | 0.148 |
| Entrapm ent Efficien cy of doxorub icin-HCl (%) | 98.27 | 95.3 | 97.47 | 94.99 | 19.55 | | 96.32 | 93.02 | 94.42 | 94.19 | 51.32 | 94.1 | 19.06 |

According to Table 5, each liposome prepared with the compositions showed a size distribution of 100 to 150 nm, exhibited a polydispersity index of 0.1, indicating a uniform size distribution. In addition, the loading efficiency of doxorubicin-HCl showed an excellent loading rate of more than 90% of the added doxorubicin volume being loaded into the liposomes. Exceptionally, only the composition 008 showed unstable particles during the manufacturing stage, aggregated in a short time, and tended to aggregate more over time. However, there was no aggregation of particles or leakage of drugs over time in other compositions. To select the most optimized composition among the liposomes, compositions 004, 005, and 013, which were expected to show excellent ultrasound responsiveness and long circulation due to their high doxorubicin encapsulation efficiency and high DOPE content, were selected as candidate compositions.

### 2-2. Analysis of Drug Release Effect by Ultrasound

Ultrasound response tests of liposomes were performed using compositions 004, 005, and 013 selected as candidate compositions. The analysis of drug release rate by ultrasound was performed using an ultrasonicator, in which the liposome containing the drug was put into the ultrasonicator and treated with ultrasound at a frequency of 24 kHz at 20% amplitude (92W/cm²) intensity for 60 seconds. The drug released from the liposome was separated by a PD-10 column and quantified by measuring the absorbance at 295 nm using UV-vis. As experimental controls, a composition 011 containing relatively less sonosensitive lipids and Doxil composition liposomes loaded with vincristine sulfate or doxorubicin-HCl, a commercial product, were used.

The loading rates of vincristine sulfate or doxorubicin-HCl and drug release rates by ultrasound for each liposome are shown in Table 6 below.

**[Table 6]**

| **Evaluation Results of Drug Loading Efficiency and Ultrasound Responsiveness According to Liposome Composition** | | | | | | |
|---|---|---|---|---|---|---|
| **Liposome** | | **004** | **005** | **011** | **013** | **Doxil** |
| **Loading efficiency(%)** | **Doxorubicin** | 98.49 | 96.48 | 94.75 | 51.80 | 93.64 |
| | **Vincristine** | 73.31 | 68.74 | 61.16 | 81.16 | 58.73 |
| **Release (%)** | **Doxorubicin** | 50.73 | 42.32 | 8.78 | 72.22 | 19.90 |
| | **Vincristine** | 66.48 | 63.48 | 66.87 | 54.46 | 56.72 |

Comparing the loading efficiencies first, for doxorubicin-HCl, compositions 004, 005, 011, and Doxil exhibited high loading efficiencies with over 90% of the added drug encapsulated. Only in the case of composition 013 was a somewhat lower loading efficiency of 51.8% observed due to liposome aggregation during the doxorubicin-HCl loading process. However, for vincristine sulfate, all compositions showed high loading efficiencies of 70 to 80%.

Drug release due to ultrasound responsiveness was evaluated by quantifying the drug released from each liposome according to ultrasound stimulation. For liposomes containing doxorubicin-HCl, it was confirmed that the drug release rate due to ultrasound responsiveness increased in the order of higher DOPE composition, thereby, the DOPE composition ratio is proportional to the ultrasound responsiveness of the liposomes. For sonosensitive liposomes containing vincristine sulfate, most liposomes showed similar levels of ultrasound responsiveness under the same ultrasound conditions.

Therefore, based on these experimental results, vincristine, a drug for treating brain tumors, was found to be most efficiently loaded, and the liposomes of composition 004 and 013 were judged as the optimal liposome compositions showing outstanding properties in terms of physical properties, drug encapsulation efficiency, and ultrasound responsiveness when loaded with doxorubicin. Especially in the case of composition 013, although the loading efficiency for doxorubicin was somewhat low, since it showed a high loading efficiency similar to other compositions for vincristine, it was included in the optimal liposome composition.

### 2-3. Confirmation of the Influence of Polysorbate 80 on the Ultrasound Responsiveness of Liposomes

In addition, to evaluate the impact of Polysorbate 80 on sonosensitive liposomes for penetrating the blood-brain barrier, the physical properties and ultrasound responsiveness of liposomes of composition 004 and 013, which have the highest release amounts of vincristine sulfate and doxorubicin-HCl, were assessed after the addition of Polysorbate 80. In the present invention, Polysorbate 80 is added as a component to increase the BBB penetration rate of the liposome formulation. Liposomes were manufactured with Polysorbate 80 included in the solvent at 0.1% (v/v), and all other processes were performed identically. The physical properties of liposomes containing Polysorbate 80 and the release rate of doxorubicin-HCl due to ultrasound responsiveness are as shown in Table 7 below.

**[Table 7]**

| | **004P** | **013P** |
|---|---|---|
| **Size (d.nm)** | 128.2 | 146.2 |
| **Loading efficiency (%)** | 95.3 | 94.1 |
| **Release (%)** | 73.9 | 65.9 |

As can be seen from Table 7, when Polysorbate 80 was added to the composition of liposomes 004 or 013, the particle size distribution was 128.2 nm and 146.2 nm, respectively, and the drug release rates due to ultrasound responsiveness were 73.9% and 65.9%, respectively. Since Polysorbate 80 was added to increase the BBB penetration rate of the liposomes, these experimental results show that Polysorbate 80 does not affect the physical properties of the liposomes before promoting the blood-brain barrier penetration of the sonosensitive liposomes.

### Example 3. Evaluation of the Brain Tumor Cell Penetration Ability of Sonosensitive Liposomes for Penetrating the Blood-brain Barrier through FACS Analysis

Experiments were conducted using the U87MG brain tumor cell line to assess the ability of liposomes to infiltrate brain tumor cell lines depending on their composition. To quantitatively analyze the effect of each liposome penetrating the cell line, FACS analysis was performed, and the sonosensitive liposomes for penetrating the blood-brain barrier used in the experiment were labeled with the fluorescent dye DiI and detected in the red wavelength range. The DiI dye exhibits fluorescence with excitation/emission wavelengths of 549/565 nm in the red wavelength range.

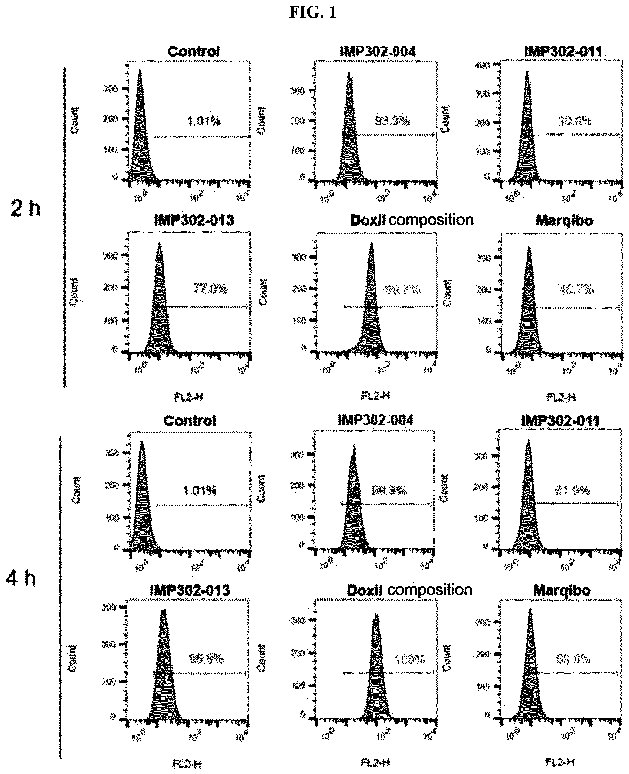

The experiment was carried out as follows. Before treating with liposomes, the U87MG cell line was cultured in a cell culture medium without serum for starvation for 1 hour. The starved cells were treated with each liposome solution at a concentration of 400 µg/ml, and after incubation with liposomes for 2 or 4 hours, FACS analysis was performed. The number of cells used was 3×10⁵. The results of the quantitative analysis of the brain tumor cell infiltration ability of the liposomes are shown in FIG. 1. The liposomes of compositions 004 and 013, which are the sonosensitive liposomes for penetrating the blood-brain barrier according to the present invention, and Doxil composition, showed high endocytosis efficiency, with most of the liposomes infiltrating into the cells within 2 hours after being treated on U87MG cells. The penetration efficiency for U87MG appeared to be high in the order of Doxil, 004, 013, and 011 liposomes.

Moreover, when the brain tumor cells and liposomes were incubated for 4 hours, the liposomes of compositions 004, 013, and Doxil showed near 100% infiltration efficiency as the cell infiltration rate increased over time, and the 011 composition liposomes also showed a continuous increase in endocytosis efficiency over time. In particular, the brain tumor cell infiltration effect of the sonosensitive liposomes according to the present invention was shown to be more than twice as high as that of the commercial liposome Marqibo used for loading the vincristine drug.

These results demonstrate that the sonosensitive liposomes for penetrating the blood-brain barrier according to the present invention can effectively penetrate into brain tumor cells and deliver drugs.

### Example 4. Verification of Cellular Penetration Ability of Sonosensitive Liposomes for Penetrating the Blood-brain Barrier Using a Confocal Fluorescence Microscope

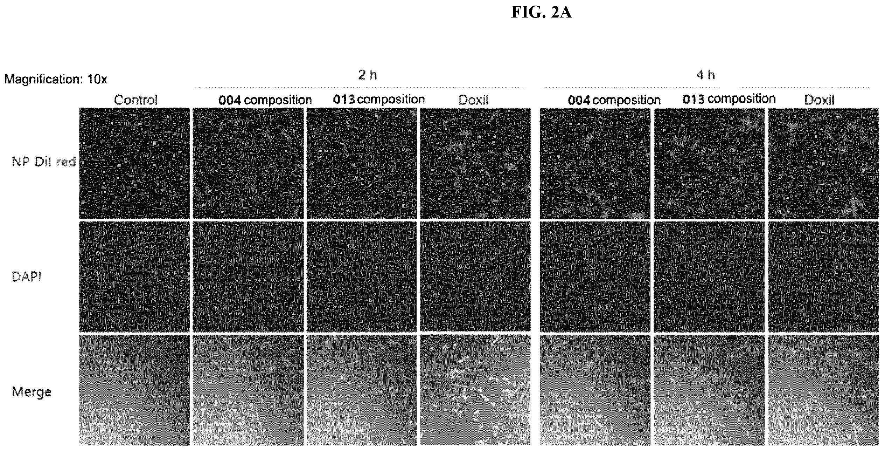

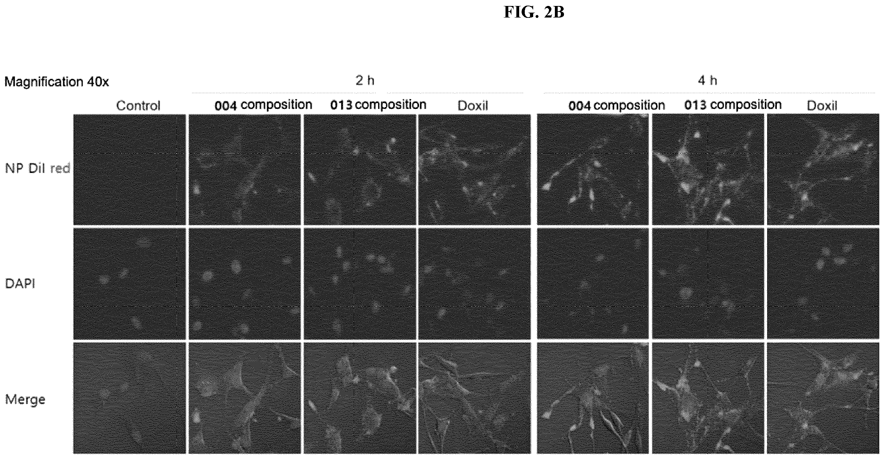

To select the optimal liposome composition for brain tumor treatment, the cellular infiltration effects over time of various compositions of liposomes treated on the U87MG cell line, a brain tumor cell line, were evaluated using a confocal fluorescence microscope. Each liposome's intracellular location was detected by labeling the liposomes with DiI fluorescent dye and illuminating them at a red wavelength. After seeding U87MG cells in a cell culture chamber, they were incubated overnight and starved for 1 hour with serum-free medium before each liposome treatment. Each liposome was treated at a concentration of 400 µg/ml to the starved cells, and after inducing cellular uptake by incubating for 2 or 4 hours, the particles were washed. Subsequently, for confocal microscope observation, the cells were fixed with 4% paraformaldehyde and then microscopy observation was performed. Imaging was conducted using a confocal fluorescence microscope to analyze the extent of brain tumor cell infiltration and the infiltration process according to the liposome composition. For fluorescence observation, liposomes labeled with DiI were photographed at a red wavelength (ex/em: 549/565 nm), and the nuclei of the cells were stained with DAPI to appear blue (ex/em: 358/461 nm). The morphology of the cells was confirmed by optical imaging with DIC. The results of observation the U87MG cell infiltration over time of the liposomes of composition 004 and 013 composition according to the present invention, and Doxil are shown in FIGs 2A and 2B.

From the results of observation the infiltration effect over time of each liposome particle in brain tumor cells using a confocal fluorescence microscope, it was found that both the liposomes of compositions 004 and 013 according to the present invention effectively penetrated into U87MG cells within 2 hours, and showed results consistent with the FACS analysis results of the Example 3. All liposomes of each composition were accurately located in the cytoplasm of U87MG cells, showed dot-shaped fluorescence, demonstrating that liposomes underwent endocytosis while maintaining the form of particles. There was no significant change when comparing the morphology of U87MG cells between the groups treated with liposomes according to the present invention or Doxil composition liposomes and the untreated control group. Such results suggest that the cellular toxicity of the sonosensitive liposomes according to the present invention is very low.

### Example 5. Verification of Brain Tumor Cell Killing Effect of Sonosensitive Liposomes for Penetrating the Blood-brain Barrier

To verify the anticancer effect of the sonosensitive liposomes according to the present invention, the anticancer effect was verified using U87MG cells, brain tumor cells. After seeding 5×10⁴ cells per well in a 96-well plate of U87MG cells, they were cultured for 24 hours. Dulbecco's Modified Eagle Medium (DMEM) containing 10% Fetal Bovine Serum (FBS) and 1% antibiotic was used as the cell culture medium. For drug efficacy evaluation, liposomes of composition 004 loaded with doxorubicin, Doxil composition liposomes, and free doxorubicin were added to the cells at various concentrations (0, 1.25, 2.5, 5, 10 µg/mL), and to compare the degree of cancer cell death depending on the presence or absence of ultrasound irradiation, liposomes irradiated with ultrasound and liposomes not treated with ultrasound were treated in designated wells. After drug treatment, it was incubated for 4 hours, and after removing all the added drugs, it was washed 3 times with phosphate buffered saline (0.01 M, pH7.4), and the degree of cancer cell proliferation was compared by culturing with the above-mentioned cell culture medium for 72 hours. The anticancer cell killing effect was quantitatively analyzed by performing an MTT analysis and measuring the absorbance at a wavelength of 570 nm.

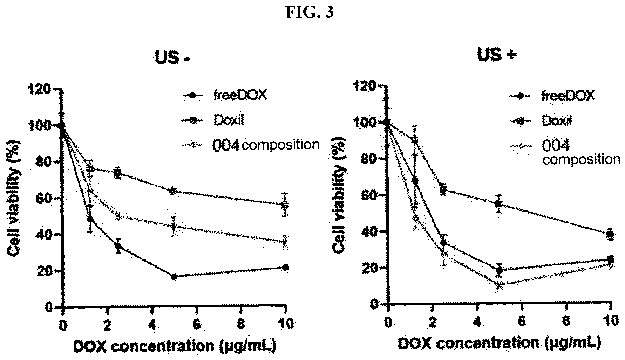

The results of verifying the anticancer cell killing effects of each liposome are shown in FIG. 3. In the case of the sonosensitive liposomes for penetrating the blood-brain barrier according to the present invention, the anticancer cell killing effect significantly increased after ultrasound treatment compared to before ultrasound treatment. On the other hand, in the case of Doxil, which does not respond to ultrasound, there was no difference in the anticancer cell killing effect before and after ultrasound treatment. In particular, the liposome of composition 004 according to the present invention showed an anticancer effect more than twice as high as that of the Doxil liposome. These results demonstrate that the sonosensitive liposomes for penetrating the blood-brain barrier according to the present invention has an excellent anticancer effect on brain tumor cells.

### Example 6. Exploration of Microbubble Conditions for Blood-Brain Barrier Opening and Comparative Analysis of Evans Blue Penetration Efficiency

To derive the optimal ultrasound conditions for opening the blood-brain barrier (BBB), the degree of opening of the blood-brain barrier was compared and analyzed using Evans blue. Evans blue is a very small dye with a molecular weight of 961 Da and is widely used for analyzing the degree of opening of the blood-brain barrier. The experiment was performed using normal BALB/c nude mice, and after injecting microbubbles via intravenous injection, ultrasound was emitted using a high-intensity focused ultrasound device, VIFU2000. After temporarily opening the blood-brain barrier through the cavitation of ultrasound and microbubbles, Evans blue (3%, w/w) was immediately injected intravenously, and the brain was extracted 24 hours later. Prior to brain extraction, the blood was discharged through normal saline perfusion to remove variables that could be caused by blood. The brain tissue was soaked in formamide, incubated overnight at 70°C to extract Evans blue in the brain tissue, and the degree of opening of the blood-brain barrier was quantitatively analyzed by measuring the absorbance at wavelengths of 670 nm and 740 nm using UV-vis.

Factors that can affect the cavitation of ultrasound and microbubbles, a mechanism that opens the blood-brain barrier, include ultrasound conditions such as the frequency, intensity, duty cycle, and ultrasound emission time of the ultrasound, and also the number of microbubbles. Therefore, to confirm the ultrasound conditions and the number of microbubbles that can open the blood-brain barrier, the degree of opening of the blood-brain barrier according to the number of microbubbles was first quantitatively analyzed. The following Table 8 represents the comparative experimental design of the degree of opening of the blood-brain barrier according to the number of microbubbles.

**[Table 8]**

| **Microbubble Condition Verification Experiment for Blood-Brain Barrier Opening** | | | | |
|---|---|---|---|---|
| **Experiment No.** | **Ultrasound** | | | **Microbubble (MB: SonoVue)** |
| | **DC, PRF** | **Time/spot** | **Power(w)** | |
| 1 | Freq. 1 MHz | 60s | 1 | 2×10⁶/g |
| 2 | DC : 5% | | | 5×10⁶/g |
| 3 | PRF : 1 Hz | | | 10×10⁶/g |

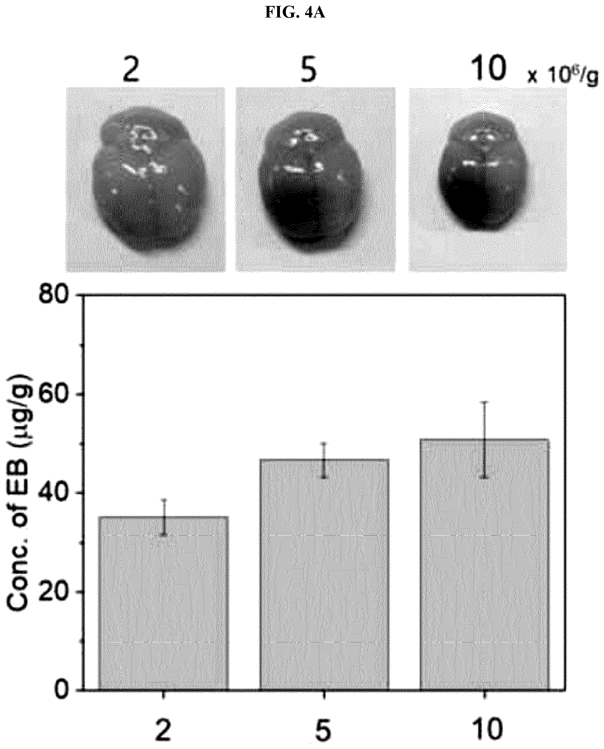

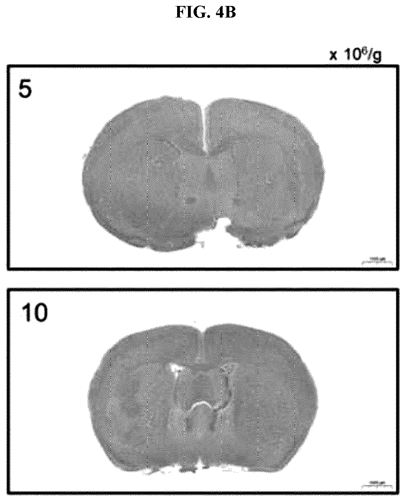

The results of analyzing the penetration efficiency of Evans blue, which penetrated the blood-brain barrier and accumulated in the brain under each opening condition in Table 8 above, are shown in FIG. 4A. As can be confirmed in the image and graph of FIG. 4A, as the number of microbubbles increased, the degree of opening of the blood-brain barrier increased, thereby improving the penetration efficiency of Evans blue. The above results suggest that as the number of microbubbles increases, the number of microbubbles resonating with ultrasound increases, resulting in an increase in cavitation, and ultimately, an increase in the penetration efficiency of the dye in the brain tissue, c

### Example 7. Exploration of Ultrasound Conditions for Blood-Brain Barrier Opening and Comparative Analysis of Evans Blue Penetration Efficiency

Following Example 6, to verify the ultrasound conditions for opening the blood-brain barrier, the penetration efficiency of Evans blue according to ultrasound conditions (intensity, frequency, and duty cycle) and ultrasound treatment time was quantitatively analyzed. Table 9 shows the comparative experimental design of the degree of opening of the blood-brain barrier according to the ultrasound conditions. The number of injected microbubbles was unified to 5×10⁶/g.

**[Table 9]**

| **Ultrasound Condition Verification Experiment for Blood-Brain Barrier Opening** | | | | |
|---|---|---|---|---|
| **Experiments No.** | **Power (W)** | **PRF (Hz)** | **Duty cycle (%)** | **Times (s)** |
| 1 | 1 | 1 | 1 | 60 |
| 2 | | | | 120 |
| 3 | | | | 180 |
| 4 | | | 5 | 60 |
| 5 | | | 10 | |
| 6 | 2 | | 1 | |
| 7 | 3 | | | |

After administering the microbubbles intravenously, the ultrasound was immediately processed, and after the ultrasound treatment was completed, 3% (w/w) Evans blue was administered intravenously. After 24 hours, the brain, completely devoid of blood through normal saline perfusion, was extracted. The quantitative analysis of Evans blue was performed in the same manner as in the aforementioned Example 5.

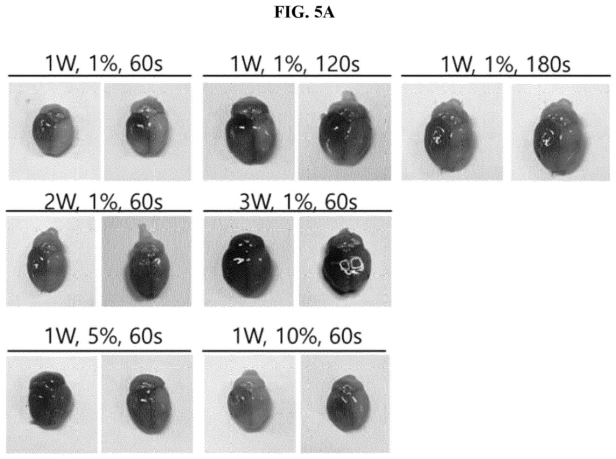

The results of the analysis of the penetration effect of Evans blue according to the ultrasound conditions are shown in FIG. 5A. As shown in the figure, as the intensity and emission time of the ultrasound increased, the overall opening effect of the blood-brain barrier increased, and the penetration effect of Evans blue also increased proportionally. Even in the experimental group treated with the lowest intensity ultrasound (Group 1), it showed a pattern of effectively penetrating Evans blue in the tissue at the ultrasound-exposed site, and it continued to increase as the exposure time increased. It also showed that the penetration effect of Evans blue increased as the intensity and duty cycle of the ultrasound increased.

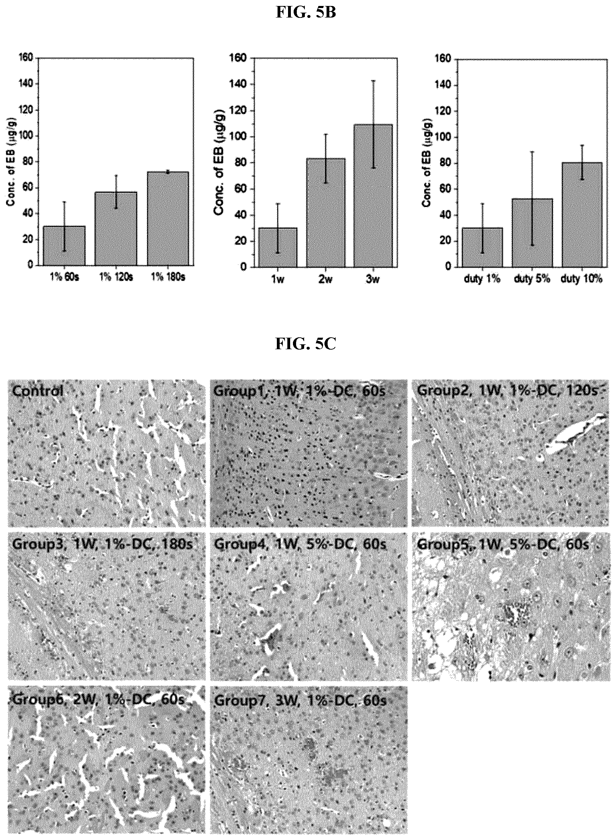

According to the quantitative analysis results, it was found that the increase in the intensity of the ultrasound rather than the emission time of the ultrasound was a factor that further improved the penetration effect of Evans blue. In addition, the increase in the duty cycle showed a similar pattern of increasing the penetration efficiency of Evans blue as increasing the ultrasound exposure time (FIG. 5B).

However, since some of the experimental groups died, in order to determine whether the penetration of Evans blue into the brain tissue was due to the temporary safe opening of the blood-brain barrier or due to brain tissue damage, additional H&E staining was performed to evaluate the stability of the brain tissue under each ultrasound condition. As a result, as shown in FIG. 5C, in the experimental group exposed to strong intensity ultrasound (Groups 6 and 7), a pattern of bleeding was observed in a considerably small area compared to the total area of the brain and the ultrasound-exposed area, but in the experimental group exposed to low-intensity ultrasound (Groups 1 to 5), no bleeding occurred. Based on the above results, it was confirmed that the safe ultrasound conditions that do not cause brain tissue damage are the ultrasound conditions of Groups 1, 2, and 6.

### Example 8. Analysis of Blood-Brain Barrier Permeability Efficiency of Sonosensitive Liposomes for Penetrating the Blood-brain Barrier

In order to analyze the blood-brain barrier penetration efficiency of the sonosensitive liposomes for penetrating the blood-brain barrier according to the present invention, the liposomes were labeled with DiD fluorescent dye that shows near-infrared (NIR) wavelengths, and fluorescence analysis was performed with an *in vivo* Imaging System (IVIS). To open the blood-brain barrier, ultrasound was emitted for 60 seconds under the conditions of 1 W, 1%-duty cycle, and 1 Hz-PRF, which are the ultrasound conditions derived through the Example 7. The opening of the blood-brain barrier was performed in the same manner as described in the Examples 6 and 7, and each liposome was immediately injected intravenously as soon as the ultrasound emission ended. After 24 hours after liposome injection, each organ was extracted and fluorescence photography was performed with IVIS. By analyzing the fluorescent images and performing a quantitative analysis of the fluorescence intensity for each organ, the liposome composition most optimal for penetrating the blood-brain barrier was explored.

Among the liposomes used in the experiment, all but the 004 composition liposome contain sphingomyelin, a representative type of sphingolipid. Sphingomyelin is known to be able to penetrate the blood-brain barrier, can circulate for a long time in the bloodstream, and is known to have a high half-life. Therefore, in order to find the optimal composition of liposomes that can effectively pass through the blood-brain barrier and accumulate in the brain, a comparative analysis of the blood-brain barrier penetration efficiency of liposomes according to the composition ratio of sphingomyelin was performed. The compositions of each liposome used in the experiment are as shown in Table 10.

**[Table 10]**

| **Composition Components and Ratios (molar ratio, %) of Liposomes used in Blood-Brain Barrier Penetration Experiments** | | | | | | |
|---|---|---|---|---|---|---|
| **Lipid** | **004** | **005** | **006** | **007** | **011** | **Marqibo** |
| DOPE | 65 | 45 | 50 | 10 | 30 | - |
| sphingomyelin | - | 45 | 50 | 90 | 70 | 60 |
| DSPE-mPEG2k | 5 | 10 | - | - | - | - |
| Cholesterol | 30 | - | - | - | - | 40 |
| DSPC | 10 | - | - | - | - | - |
| lyso-PC | 5 | - | - | - | - | - |

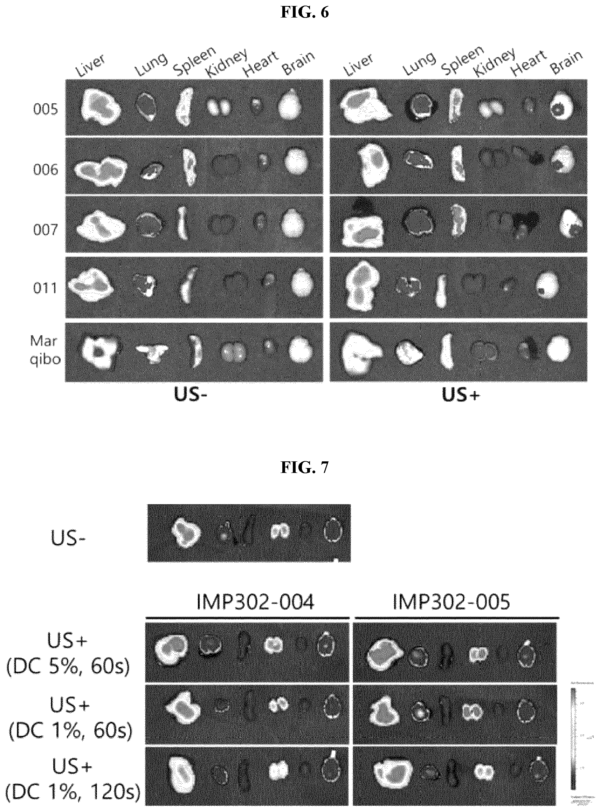

The liposomes prepared in accordance with the compositions in Table 10 were each injected into normal mice, and ultrasound was emitted under the safe conditions for brain tissue confirmed in Example 7, namely, 1 W, 1% DC, and 60s of ultrasound parameters. The blood-brain barrier penetration efficiency and organ distribution results for each liposome are shown in FIG. 6.

Upon analysis of organ distribution patterns, all liposomes used in the present study showed a common tendency to accumulate most in the liver and were also found to accumulate at a considerable level in the spleen. These results are considered due to the fact that the liposomes, having a size of approximately 100 to 200 nm, are recognized as foreign substances in the liver and expelled from the body. Furthermore, it has also been reported in previous studies that DOPE tends to accumulate in the spleen.

Observation of the brain in the group not subjected to ultrasound emission revealed that liposomes of all compositions were not detected. In other words, it appeared that the penetration of liposomes into brain tissue was inhibited by the blood-brain barrier. This is generally because the blood-brain barrier actively permits the passage of only small molecules, so liposomes, despite the penetration function of sphingomyelin, with a large size of about 100 to 200 nm, cannot pass through the blood-brain barrier.

In contrast, when opening of the blood-brain barrier was induced using ultrasound and microbubbles, liposomes were detected in the part exposed to ultrasound, regardless of the composition of the liposome. There was no particular observation of the trend of increase in the delivery efficiency to brain tissue with increasing content of sphingomyelin. Compared with the known blood-brain barrier penetration effect of sphingomyelin, the liposome according to the present invention showed a pattern about 2 to 3 times larger. It is possible for liposomes of about 20 to 50 nm in size to penetrate the blood-brain barrier, but liposomes reaching 100 nm are not easy to penetrate the blood-brain barrier, and to overcome this, when the opening of the blood-brain barrier is induced, it is determined that the opened path of the blood-brain barrier should show a gap of more than 100 nm.

Additionally, to verify the influence of sphingomyelin on the blood-brain barrier penetration effect of liposomes, a comparative analysis was first made of the 004 composition liposome, which did not include sphingomyelin, and the 005 composition liposome, which included other sphingomyelin, and it was confirmed that both penetrated the blood-brain barrier at a similar level (FIG. 7).

Furthermore, a comparative experiment was conducted for the 004 and 005 composition liposomes by setting the duty cycle among the ultrasound parameters to 1 or 5%. Other parameters were set to the safe ultrasound parameters confirmed in Example 7. As a result, as can be seen in FIG. 7, it appeared that the 5% duty cycle improved the penetration efficiency of both liposomes more than the 1% duty cycle. Also, as the duration time increased, the fluorescence intensity increased, indicating improved blood-brain barrier penetration effects of the liposomes. The above results show that as the duty cycle and ultrasound duration time increase, the blood-brain barrier penetration rate of liposomes can be further increased.

### Example 9. Comparison of Blood-Brain Barrier Penetration Effect of Sonosensitive Liposome for Penetrating the Blood-brain Barrier and Doxil Liposome

In order to compare the delivery effect to the brain of the sonosensitive liposome for penetrating the blood-brain barrier according to the present invention and the non-responsive liposome Doxil, a comparison experiment was conducted by manufacturing liposomes with the 004 composition and Doxil composition. The experimental method was performed in the same manner as the example, and the degree of brain tissue delivery was quantitatively analyzed by measuring the fluorescence intensity with IVIS.

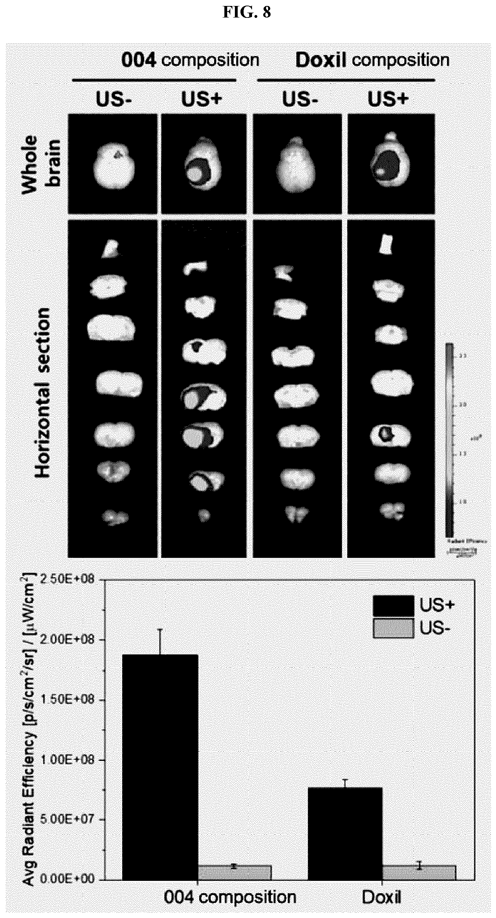

The experimental results are shown in FIG. 8. Both the sonosensitive liposome (004 composition liposome) according to the present invention and the non-responsive liposome Doxil detected fluorescence at the site where the ultrasound was emitted, confirming that both types of liposomes penetrated the blood-brain barrier due to the cavitation of ultrasound and microbubbles. However, in the case of the sonosensitive liposome according to the present invention, it was found that the delivery effect to the brain was nearly twice as high compared to the Doxil liposome. The results show that the sonosensitive liposome for penetrating the blood-brain barrier according to the present invention not only has excellent ultrasound responsiveness but also higher penetration effect to brain tissue, and can be more effectively delivered to brain tissue.

The description of the present invention as stated above is for illustrative purposes only, and a person having ordinary skill in the art to which the present invention pertains will understand that the technical idea or essential characteristics of the present invention can be easily modified into other specific forms without changing them. Therefore, the examples described above should be understood as illustrative in all aspects and not limited.

### [Industrial Applicability]

The present invention relates to sonosensitive liposomes for penetrating the blood-brain barrier, and the sonosensitive liposomes can effectively penetrate the blood-brain barrier when stimulated by ultrasound, in addition to having outstanding drug encapsulation efficiency and drug release effect by ultrasound. In particular, the sonosensitive liposomes according to the present invention can circulate for a long time in the body, thus it has excellent efficiency in penetrating the blood-brain barrier, and has a high affinity for brain tumor cells, thus the delivery effect to the tumor site is outstanding. Therefore, the sonosensitive liposomes according to the present invention can be used as a drug delivery carrier for delivering a therapeutic agent for brain disease, among others, to the brain. In particular, the inventors have identified optimal cavitation conditions that can stably open the blood-brain barrier to further enhance the drug delivery effect of the sonosensitive liposomes, and it is expected that excellent therapeutic effects can be obtained in various brain diseases when these cavitation conditions are combined with the sonosensitive liposomes for penetrating the blood-brain barrier according to the present invention.

## Claims

1. A composition for penetrating the blood-brain barrier, comprising sonosensitive liposomes comprising DSPC (1,2-distearoyl-sn-glycero-3-phosphocholine), DSPE-mPEG2000 (1,2-Distearoyl-sn-glycero-3-phosphoethanolamine-N-[methoxy(polyethylene glycol)-2000]), DOPE (1,2-Dioleoyl-sn-glycero-3-phosphoethanolamine), cholesterol, and lyso-PC (lysophosphatidylcholine) as active ingredients.

2. The composition for penetrating the blood-brain barrier of claim 1, wherein the DSPC, the DSPE-mPEG2000, the DOPE, the cholesterol, and the lyso-PC are comprised at molar ratio (mole%) of 1-50 : 1-10 : 5~80 : 0.1-50 : 0.1-20 respectively.

3. The composition for penetrating the blood-brain barrier of claim 1,
wherein the DSPC is comprised at dry weight percentages of 0.1 to 50 based on the total liposomes,
the DSPE-mPEG2000 is comprised at dry weight percentages of 3 to 50 based on the total liposomes,
the DOPE is comprised at dry weight percentages of 1 to 80 based on the total liposomes,
the cholesterol is comprised at dry weight percentages of 0.05 to 40 based on the total liposomes, and
the lyso-PC are comprised at dry weight percentages of 0.5 to 10 based on the total liposomes.

4. The composition for penetrating the blood-brain barrier of claim 1, wherein the sonosensitive liposomes for penetrating the blood-brain barrier further comprise at least one selected from a group consisting of sphingolipid and polysorbate.

5. A composition for penetrating the blood-brain barrier, comprising sonosensitive liposomes comprising DOPE (1,2-Dioleoyl-sn-glycero-3-phosphoethanolamine) and sphingolipid as active ingredients.

6. The composition for penetrating the blood-brain barrier of claim 5, wherein the DOPE is comprised at a molar ratio (mole%) of 5 to 80 based on the total liposomes, and the sphingolipid is comprised at a molar ratio (mole%) of 5 to 80 based on the total liposomes.

7. The composition for penetrating the blood-brain barrier of claim 5, wherein the sonosensitive liposomes further comprise DSPE-mPEG2000.

8. The composition for penetrating the blood-brain barrier of claim 7, wherein the DSPE-mPEG2000 is comprised at a molar ratio (mole%) of 1 to 20, based on the total liposomes.

9. The composition for penetrating the blood-brain barrier of claim 1, wherein the sonosensitive liposome is at least one selected from the group consisting of:
(a) having a particle size of 100 to 200 nm; and
(b) the ratio of drug encapsulated in the liposomes to the total drug added is 50-100%.

10. The composition for penetrating the blood-brain barrier of claim 1, wherein the sonosensitive liposomes penetrate the blood-brain barrier when exposed to ultrasound.

11. The composition for penetrating the blood-brain barrier of claim 1, wherein the composition is for delivering a drug into the brain.

12. The composition for penetrating the blood-brain barrier of claim 11, wherein the drug is a therapeutic agent for brain disease.

13. The composition for penetrating the blood-brain barrier of claim 12, wherein the brain disease is at least one selected from a group consisting of brain tumors, brain infections caused by bacteria or viruses, Parkinson's disease, encephalitis, stroke, palsy, Alzheimer's, Lou Gehrig's disease, Huntington's disease, Pick's disease, Creutzfeldt-Jakob disease, epilepsy, thrombosis, embolism, brain infarction, palsy, small artery occlusion, and cerebral metabolic disorders.

14. The composition for penetrating the blood-brain barrier of claim 12, wherein the therapeutic agent for brain disease is at least one selected from a group consisting of vincristine, vinblastine, vinflunine, vindesine, vinorelbine, temozolomide, carmustine, lomustine, cabazitaxel, docetaxel, larotaxel, ortataxel, paclitaxel, tesetaxel, ixabepilone, lomustine, procarbazine, rituximab, tocilizumab, temozolomide, carboplatin, erlotinib, irinotecan, enzastaurin, vorinostat, doxorubicin, cisplatin, Gleevec, 5-fluorouracil, tamoxifen, topotecan, belotecan, imatinib, floxuridine, gemcitabine, leuprolide, flutamide, zoledronate, methotrexate, camptothecin, hydroxyurea, streptozocin, valrubicin, retinoic acid, mechlorethamine, chlorambucil, busulfan, doxifluridine, mitomycin, prednisone, Afinitor, mitoxantrone, levodopa, carbidopa, entacapone, tolcapone, dopamine agonists, donepezil, galantamine, rivastigmine, memantine, anticholinergics, and amantadine.

15. The composition for penetrating the blood-brain barrier of claim 1, wherein the sonosensitive liposomes are hydrated with ammonium sulfate, ammonium citrate, or TEA-SOS.

16. The composition for penetrating the blood-brain barrier of claim 1, wherein the composition is administered sequentially or simultaneously with ultrasound treatment.

17. The composition for penetrating the blood-brain barrier of claim 16, wherein the ultrasound is at least one selected from a group consisting of:
(a) the frequency of the ultrasound is 20 kHz to 3 MHz; and
(b) the duty cycle is 0.5 to 20%.

18. The composition for penetrating the blood-brain barrier of claim 16, wherein the ultrasound treatment is performed sequentially or simultaneously with the administration of microbubble.

19. A drug delivery carrier for penetrating the blood-brain barrier, comprising sonosensitive liposomes comprising DSPC (1,2-distearoyl-sn-glycero-3-phosphocholine), DSPE-mPEG2000 (1,2-Distearoyl-sn-glycero-3-phosphoethanolamine-N-[methoxy(polyethylene glycol)-2000]), DOPE (1,2-Dioleoyl-sn-glycero-3-phosphoethanolamine), cholesterol, and lyso-PC (lysophosphatidylcholine) as active ingredients.

20. A method for manufacturing the composition for penetrating the blood-brain barrier of claim 1, comprising the following steps:
(S1) dissolving at least one selected from a group consisting of DSPC, DSPE-mPEG2000, DOPE, cholesterol, and lyso-PC in a first organic solvent;
(S2) evaporating the organic solvent to manufacture liposome film; and
(S3) hydrating the liposome film with an aqueous solution.

21. The method of claim 20, wherein the first organic solvent is at least one selected from a group consisting of dimethylacetamide, dimethylformamide, dimethylsulfoxide, chloroform, methanol, ethanol, and ether.

22. The method of claim 20, wherein polysorbate is added to the first organic solvent in step (S1).

23. A method of delivering drugs to the brain by administering an effective dose of drug-containing sonosensitive liposomes to a subject in need thereof, wherein the sonosensitive liposomes comprise DSPC, DSPE-mPEG2000, DOPE, cholesterol, and lyso-PC.

24. Use of sonosensitive liposomes comprising DSPC, DSPE-mPEG2000, DOPE, cholesterol, and lyso-PC for the manufacture of a drug delivery carrier targeting the brain.

25. Use of sonosensitive liposomes comprising DSPC, DSPE-mPEG2000, DOPE, cholesterol, and lyso-PC for drug delivery to the brain.
